# EUROPÄISCHE PATENTSCHRIFT

(11) **EP 0 805 204 B1**
(45) Veröffentlichungstag und Bekanntmachung des Hinweises auf die Patenterteilung: **29.11.2006**
(21) Anmeldenummer: 97250143.1
(22) Anmeldetag: 28.04.1997
(51) Int. Cl.: C12N 15/12, C07K 14/705, C07K 16/28, A61K 38/17, A61K 39/395, C12Q 1/68, A61K 31/70, G01N 33/50

(54) **Nebenhoden-spezifisches Rezeptorprotein und dessen Verwendung**
Epididymis-specific receptor protein and its use
Protéine récepteur spécifique de l'épididyme et son utilisation

(30) Priorität: 29.04.1996 DE 19617940
(43) Veröffentlichungstag der Anmeldung: 05.11.1997
(73) Patentinhaber: Schering Aktiengesellschaft, 13353 Berlin (DE)
(72) Erfinder: Osterhoff, Caroline, Dr., 20255 Hamburg (DE); Ivell, Richard, Dr., 20257 Hamburg (DE)

(56) Entgegenhaltungen:
- EP-A- 0 440 321
- DATABASE EMBL DATABASE RELEASE 47 [Online] accession no. HS478342, 13. Mai 1996 (1996-05-13) XP002180832
- OSTERHOFF ET AL.: DNA AND CELL BIOLOGY , Bd. 16, Nr. 4, 1997, Seiten 379-89, XP002925525
- KIRCHLOF C ET AL: "Cloning and analysis of mRNAs expressed specifically in the human epididymis" INTERNATIONAL JOURNAL OF ANDROLOGY, BLACKWELL SCIENTIFIC PUBLICATIONS, OXFORD, GB, Bd. 2, Nr. 13, 1. April 1990 (1990-04-01), Seiten 155-167, XP002075040 ISSN: 0105-6263
- OSTERHOFF C. ET AL.: "Molecular cloning and characterization of a novel human sperm antigen (HE2) specifically expressed in the proximal epididymis" BIOLOGY OF REPRODUCTION, Bd. 50, 1994, Seiten 516-525,

### SEQUENZPROTOKOLL

(1) ALLGEMEINE INFORMATION:
   (i) ANMELDER:
      (A) NAME: Institut für Hormon- und Fortpflanzungsforschung GmbH
      (B) STRASSE: Grandweg 64
      (C) ORT: Hamburg
      (E) LAND: Deutschland
      (F) POSTLEITZAHL: 22529
   (ii) ANMELDETITEL: Nebenhoden-spezifisches Rezeptorprotein
   (iii) ANZAHL DER SEQUENZEN: 2
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) LÄNGE: 4665 Basenpaare
      (B) ART: Nukleinsäure
      (C) STRANGFORM: Einzel
      (D) TOPOLOGIE: linear
   (ii) ART DES MOLEKÜLS: cDNS
   (ix) MERKMALE:
      (A) NAME/SCHLÜSSEL: CDS
      (B) LAGE: 1..3114
   (ix) MERKMALE:
      (A) NAME/SCHLÜSSEL: 3'UTR
      (B) LAGE: 3115..4665
   (ix) MERKMALE:
      (A) NAME/SCHLÜSSEL: polyA-site
      (B) LAGE: 4647..4652
   (xi) SEQUENZBESCHREIBUNG: SEQ ID NO: 1:
   AGC CAG CCC GAG GAC GCG AGC GGC AGG TGT GCA CAG AGG TTC TCC ACT      48
   Ser Gln Pro Glu Asp Ala Ser Gly Arg Cys Ala Gln Arg Phe Ser Thr
     1               5                  10                  15
   TTG TTT TCT GAA CTC GCG GTC AGG ATG GTT TTC TCT GTC AGG CAG TGT 96
   Leu Phe Ser Glu Leu Ala Val Arg Met Val Phe Ser Val Arg Gln Cys
   20 25 30
   GGC CAT GTT GGC AGA ACT GAA GAA GTT TTA CTG ACG TTC AAG ATA TTC 144
   Gly His Val Gly Arg Thr Glu Glu Val Leu Leu Thr Phe Lys Ile Phe
   35 40 45
   CTT GTC ATC ATT TGT CTT CAT GTC GTT CTG GTA ACA TCC CTG GAA GAA 192
   Leu Val Ile Ile Cys Leu His Val Val Leu Val Thr Ser Leu Glu Glu
   50 55 60
   GAT ACT GAT AAT TCC AGT TTG TCA CCA CCA CCT GCT AAA TTA TCT GTT 240
   Asp Thr Asp Asn Ser Ser Leu Ser Pro Pro Pro Ala Lys Leu Ser Val
   65 70 75 80
   GTC AGT TTT GCC CCC TCC TCC AAT GAG GTT GAA ACA ACA AGC CTC AAT 288
   Val Ser Phe Ala Pro Ser Ser Asn Glu Val Glu Thr Thr Ser Leu Asn
   85 90 95
   GAT GTT ACT TTA AGC TTA CTC CCT TCA AAC GAA ACA GAA AAA ACT AAA 336
   Asp Val Thr Leu Ser Leu Leu Pro Ser Asn Glu Thr Glu Lys Thr Lys
   100 105 110
   ATC ACT ATA GTA AAA ACC TTC AAT GCT TCA GGC GTC AAA CCC CAG AGA 384
   Ile Thr Ile Val Lys Thr Phe Asn Ala Ser Gly Val Lys Pro Gln Arg
   115 120 125
   AAT ATC TGC AAT TTG TCA TCT ATT TGC AAT GAC TCA GCA TTT TTT AGA 432
   Asn Ile Cys Asn Leu Ser Ser Ile Cys Asn Asp Ser Ala Phe Phe Arg
   130 135 140
   GGT GAG ATC ATG TTT CAA TAT GAT AAA GAA AGC ACT GTT CCC CAG AAT 480
   Gly Glu Ile Met Phe Gln Tyr Asp Lys Glu Ser Thr Val Pro Gln Asn
   145 150 155 160
   CAA CAT ATA ACG AAT GGC ACC TTA ACT GGA GTC CTG TCT CTA AGT GAA 528
   Gln His Ile Thr Asn Gly Thr Leu Thr Gly Val Leu Ser Leu Ser Glu
   165 170 175
   TTA AAA CGC TCA GAG CTC AAC AAA ACC CTG CAA ACC CTA AGT GAG ACT 576
   Leu Lys Arg Ser Glu Leu Asn Lys Thr Leu Gln Thr Leu Ser Glu Thr
   180 185 190
   TAC TTT ATA ATG TGT GCT ACA GCA GAG GCC CAA AGC ACA TTA AAT TGT 624
   Tyr Phe Ile Met Cys Ala Thr Ala Glu Ala Gln Ser Thr Leu Asn Cys
   195 200 205
   ACA TTC ACA ATA AAA CTG AAT AAT ACA ATG AAT GCA TGT GCT GCA ATA 672
   Thr Phe Thr Ile Lys Leu Asn Asn Thr Met Asn Ala Cys Ala Ala Ile
   210 215 220
   GCC GCT TTG GAA AGA GTA AAG ATT CGA CCA ATG GAA CAC TGC TGC TGT    720
   Ala Ala Leu Glu Arg Val Lys Ile Arg Pro Met Glu His Cys Cys Cys
   225                 230                 235                 240
   TCT GTC AGG ATA CCC TGC CCT TCC TCC CCA GAA GAG TTG GGA AAG CTT    768
   Ser Val Arg Ile Pro Cys Pro Ser Ser Pro Glu Glu Leu Gly Lys Leu
                   245                 250                 255
   CAG TGT GAC CTG CAG GAT CCC ATT GTC TGT CTT GCT GAC CAT CCA CGT    816
   Gln Cys Asp Leu Gln Asp Pro Ile Val Cys Leu Ala Asp His Pro Arg
               260                 265                 270
   GGC CCA CCA TTT TCT TCC AGC CAA TCC ATC CCA GTG GTG CCT CGG GCC    864
   Gly Pro Pro Phe Ser Ser Ser Gln Ser Ile Pro Val Val Pro Arg Ala
           275                 280                 285
   ACT GTG CTT TCC CAG GTC CCC AAA GCT ACC TCT TTT GCT GAG CCT CCA    912
   Thr Val Leu Ser Gln Val Pro Lys Ala Thr Ser Phe Ala Glu Pro Pro
       290                 295                 300
   GAT TAT TCA CCT GTG ACC CAC AAT GTT CCC TCT CCA ATA GGG GAG ATT    960
   Asp Tyr Ser Pro Val Thr His Asn Val Pro Ser Pro Ile Gly Glu Ile
   305                 310                 315                 320
   CAA CCC CTT TCA CCC CAG CCT TCA GCT CCC ATA GCT TCC AGC CCT GCC    1008
   Gln Pro Leu Ser Pro Gln Pro Ser Ala Pro Ile Ala Ser Ser Pro Ala
                   325                 330                 335
   ATT GAC ATG CCC CCA CAG TCT GAA ACG ATC TCT TCC CCT ATG CCC CAA    1056
   Ile Asp Met Pro Pro Gln Ser Glu Thr Ile Ser Ser Pro Met Pro Gln
               340                 345                 350
   ACC CAT GTC TCC GGC ACC CCA CCT CCT GTG AAA GCC TCA TTT TCC TCT    1104
   Thr His Val Ser Gly Thr Pro Pro Pro Val Lys Ala Ser Phe Ser Ser
           355                 360                 365
   CCC ACC GTG TCT GCC CCT GCG AAT GTC AAC ACT ACC AGC GCA CCT CCT    1152
   Pro Thr Val Ser Ala Pro Ala Asn Val Asn Thr Thr Ser Ala Pro Pro
       370                 375                 380
   GTC CAG ACA GAC ATC GTC AAC ACC AGC AGT ATT TCT GAT CTT GAG AAC    1200
   Val Gln Thr Asp Ile Val Asn Thr Ser Ser Ile Ser Asp Leu Glu Asn
   385                 390                 395                 400
   CAA GTG TTG CAG ATG GAG AAG GCT CTG TCC TTG GGC AGC CTG GAG CCT    1248
   Gln Val Leu Gln Met Glu Lys Ala Leu Ser Leu Gly Ser Leu Glu Pro
                   405                 410                 415
   AAC CTC GCA GGA GAA ATG ATC AAC CAA GTC AGC AGA CTC CTT CAT TCC    1296
   Asn Leu Ala Gly Glu Met Ile Asn Gln Val Ser Arg Leu Leu His Ser
               420                 425                 430
   CCG CCT GAC ATG CTG GCC CCT CTG GCT CAA AGA TTG CTG AAA GTA GTG        1344
   Pro Pro Asp Met Leu Ala Pro Leu Ala Gln Arg Leu Leu Lys Val Val
           435                 440                 445
   GAT GAC ATT GGC CTA CAG CTG AAC TTT TCA AAC ACG ACT ATA AGT CTA        1392
   Asp Asp Ile Gly Leu Gln Leu Asn Phe Ser Asn Thr Thr Ile Ser Leu
       450                 455                 460
   ACC TCC CCT TCT TTG GCT CTG GCT GTG ATC AGA GTG AAT GCC AGT AGT        1440
   Thr Ser Pro Ser Leu Ala Leu Ala Val Ile Arg Val Asn Ala Ser Ser
   465                 470                 475                 480
   TTC AAC ACA ACT ACC TTT GTG GCC CAA GAC CCT GCA AAT CTT CAG GTT        1488
   Phe Asn Thr Thr Thr Phe Val Ala Gln Asp Pro Ala Asn Leu Gln Val
                   485                 490                 495
   TCT CTG GAA ACC CAA GCT CCT GAG AAC AGT ATT GGC ACA ATT ACT CTT        1536
   Ser Leu Glu Thr Gln Ala Pro Glu Asn Ser Ile Gly Thr Ile Thr Leu
               500                 505                 510
   CCT TCA TCG CTG ATG AAT AAT TTA CCA GCT CAT GAC ATG GAG CTA GCT        1584
   Pro Ser Ser Leu Met Asn Asn Leu Pro Ala His Asp Met Glu Leu Ala
           515                 520                 525
   TCC AGG GTT CAG TTC AAT TTT TTT GAA ACA CCT GCT TTG TTT CAG GAT        1632
   Ser Arg Val Gln Phe Asn Phe Phe Glu Thr Pro Ala Leu Phe Gln Asp
       530                 535                 540
   CCT TCC CTG GAG AAC CTC TCT CTG ATC AGC TAC GTC ATA TCA TCG AGT        1680
   Pro Ser Leu Glu Asn Leu Ser Leu Ile Ser Tyr Val Ile Ser Ser Ser
   545                 550                 555                 560
   GTT GCA AAC CTG ACC GTC AGG AAC TTG ACA AGA AAC GTG ACA GTC ACA        1728
   Val Ala Asn Leu Thr Val Arg Asn Leu Thr Arg Asn Val Thr Val Thr
                   565                 570                 575
   TTA AAG CAC ATC AAC CCG AGC CAG GAT GAG TTA ACA GTG AGA TGT GTA        1776
   Leu Lys His Ile Asn Pro Ser Gln Asp Glu Leu Thr Val Arg Cys Val
               580                 585                 590
   TTT TGG GAC TTG GGC AGA AAT GGT GGC AGA GGA GGC TGG TCA GAC AAT        1824
   Phe Trp Asp Leu Gly Arg Asn Gly Gly Arg Gly Gly Trp Ser Asp Asn
           595                 600                 605
   GGC TGC TCT GTC AAA GAC AGG AGA TTG AAT GAA ACC ATC TGT ACC TGT        1872
   Gly Cys Ser Val Lys Asp Arg Arg Leu Asn Glu Thr Ile Cys Thr Cys
       610                 615                 620
   AGC CAT CTA ACA AGC TTC GGC GTT CTG CTG GAC CTA TCT AGG ACA TCT        1920
   Ser His Leu Thr Ser Phe Gly Val Leu Leu Asp Leu Ser Arg Thr Ser
   625                 630                 635                 640
   GTG CTG CCT GCT CAA ATG ATG GCT CTG ACG TTC ATT ACA TAT ATT GGT     1968
   Val Leu Pro Ala Gln Met Met Ala Leu Thr Phe Ile Thr Tyr Ile Gly
               645                 650                 655
   TGT GGG CTT TCA TCA ATT TTT CTG TCA GTG ACT CTT GTA ACC TAC ATA     2016
   Cys Gly Leu Ser Ser Ile Phe Leu Ser Val Thr Leu Val Thr Tyr Ile
           660                 665                 670
   GCT TTT GAA AAG ATC CGG AGG GAT TAC CCT TCC AAA ATC CTC ATC CAG     2064
   Ala Phe Glu Lys Ile Arg Arg Asp Tyr Pro Ser Lys Ile Leu Ile Gln
       675                 680                 685
   CTG TGT GCT GCT CTG CTT CTG CTG AAC CTG GTC TTC CTC CTG GAC TCG     2112
   Leu Cys Ala Ala Leu Leu Leu Leu Asn Leu Val Phe Leu Leu Asp Ser
   690                 695                 700
   TGG ATT GCT CTG TAT AAG ATG CAA GGC CTC TGC ATC TCA GTG GCT GTA     2160
   Trp Ile Ala Leu Tyr Lys Met Gln Gly Leu Cys Ile Ser Val Ala Val
   705                 710                 715                 720
   TTT CTT CAT TAT TTT CTC TTG GTC TCA TTC ACA TGG ATG GGC CTA GAA     2208
   Phe Leu His Tyr Phe Leu Leu Val Ser Phe Thr Trp Met Gly Leu Glu
                   725                 730                 735
   GCA TTC CAT ATG TAC CTG GCC CTT GTC AAA GTA TTT AAT ACT TAC ATC     2256
   Ala Phe His Met Tyr Leu Ala Leu Val Lys Val Phe Asn Thr Tyr Ile
               740                 745                 750
   CGA AAA TAC ATC CTT AAA TTC TGC ATT GTC GGT TGG GGG GTA CCA GCT     2304
   Arg Lys Tyr Ile Leu Lys Phe Cys Ile Val Gly Trp Gly Val Pro Ala
           755                 760                 765
   GTG GTT GTG ACC ATC ATC CTG ACT ATA TCC CCA GAT AAC TAT GGG CTT     2352
   Val Val Val Thr Ile Ile Leu Thr Ile Ser Pro Asp Asn Tyr Gly Leu
       770                 775                 780
   GGA TCC TAT GGG AAA TTC CCC AAT GGT TCA CCG GAT GAC TTC TGC TGG     2400
   Gly Ser Tyr Gly Lys Phe Pro Asn Gly Ser Pro Asp Asp Phe Cys Trp
   785                 790                 795                 800
   ATC AAC AAC AAT GCA GTA TTC TAC ATT ACG GTG GTG GGA TAT TTC TGT     2448
   Ile Asn Asn Asn Ala Val Phe Tyr Ile Thr Val Val Gly Tyr Phe Cys
                   805                 810                 815
   GTG ATA TTT TTG CTG AAC GTC AGC ATG TTC ATT GTG GTC CTG GTT CAG     2496
   Val Ile Phe Leu Leu Asn Val Ser Met Phe Ile Val Val Leu Val Gln
               820                 825                 830
   CTC TGT CGA ATT AAA AAG AAG AAG CAA CTG GGA GCC CAG CGA AAA ACC     2544
   Leu Cys Arg Ile Lys Lys Lys Lys Gln Leu Gly Ala Gln Arg Lys Thr
           835                 840                 845
   AGT ATT CAA GAC CTC AGG AGT ATC GCT GGC CTT ACA TTT TTA CTG GGA    2592
   Ser Ile Gln Asp Leu Arg Ser Ile Ala Gly Leu Thr Phe Leu Leu Gly
       850                 855                 860
   ATA ACT TGG GGC TTT GCC TTC TTT GCC TGG GGA CCA GTT AAC GTG ACC    2640
   Ile Thr Trp Gly Phe Ala Phe Phe Ala Trp Gly Pro Val Asn Val Thr
   865                 870                 875                 880
   TTC ATG TAT CTG TTT GCC ATC TTT AAT ACC TTA CAA GGA TTT TTC ATA    2688
   Phe Met Tyr Leu Phe Ala Ile Phe Asn Thr Leu Gln Gly Phe Phe Ile
                   885                 890                 895
   TTC ATC TTT TAC TGT GTG GCC AAA GAA AAT GTC AGG AAG CAA TGG AGG    2736
   Phe Ile Phe Tyr Cys Val Ala Lys Glu Asn Val Arg Lys Gln Trp Arg
               900                 905                 910
   CGG TAT CTT TGT TGT GGA AAG TTA CGG CTG GCT GAA AAT TCT GAC TGG    2784
   Arg Tyr Leu Cys Cys Gly Lys Leu Arg Leu Ala Glu Asn Ser Asp Trp
           915                 920                 925
   AGT AAA ACT GCT ACT AAT GGT TTA AAG AAG CAG ACT GTA AAC CAA GGA    2832
   Ser Lys Thr Ala Thr Asn Gly Leu Lys Lys Gln Thr Val Asn Gln Gly
       930                 935                 940
   GTG TCC AGC TCT TCA AAT TCC TTA CAG TCA AGC AGT AAC TCC ACT AAC    2880
   Val Ser Ser Ser Ser Asn Ser Leu Gln Ser Ser Ser Asn Ser Thr Asn
   945                 950                 955                 960
   TCC ACC ACA CTG CTA GTG AAT AAT GAT TGC TCA GTA CAC GCA AGC GGG    2928
   Ser Thr Thr Leu Leu Val Asn Asn Asp Cys Ser Val His Ala Ser Gly
                   965                 970                 975
   AAT GGA AAT GCT TCT ACA GAG AGG AAT GGG GTC TCT TTT AGT GTT CAG    2976
   Asn Gly Asn Ala Ser Thr Glu Arg Asn Gly Val Ser Phe Ser Val Gln
               980                 985                 990
   AAT GGA GAT GTG TGC CTT CAC GAT TTC ACT GGA AAA CAG CAC ATG TTT    3024
   Asn Gly Asp Val Cys Leu His Asp Phe Thr Gly Lys Gln His Met Phe
           995                 1000                1005
   AAC GAG AAG GAA GAT TCC TGC AAT GGG AAA GGC CGT ATG GCT CTC AGA    3072
   Asn Glu Lys Glu Asp Ser Cys Asn Gly Lys Gly Arg Met Ala Leu Arg
       1010                1015                1020
   AGG ACT TCA AAG CGG GGA AGC TTA CAC TTT ATT GAG CAA ATG            3114
   Arg Thr Ser Lys Arg Gly Ser Leu His Phe Ile Glu Gln Met
   1025                1030                1035
   TGATTCCTTT CTTCTAAAAT CAAAGCATGA TGCTTGACAG TGTGAAATGT CCAATTTTAC  3174
   CTTTTACACA ATGTGAGATG TATGAAAATC AACTCATTTT ATTCTCGGCA ACATCTGGAG  3234
   AAGCATAAGC TAATTAAGGG CGATGATTAT TATTACAAGA AGAAACCAAG ACATTACACC  3294
   ATGGTTTTTA GACATTTCTG ATTTGGTTTC TTATCTTTCA TTTTATAAGA AGGTTGGTTT 3354
   TAAACAATAC ACTAAGAATG ACTCCTATAA AGAAAACAAA AAAAGGTAGT GAACTTTCAG 3414
   CTACCTTTTA AAGAGGCTAA GTTATCTTTG ATAACATCAT ATAAAGCAAC TGTTGACTTC 3474
   AGCCTGTTGG TGAGTTTAGT TGTGCATGCC TTTGTTGTAT ATAAGCTAAA TTCTAGTGAC 3534
   CCATGTGTCA AAAATCTTAC TTCTACATTT TTTTGTATTT ATTTTCTACT GTGTAAATGT 3594
   ATTCCTTTGT AGAATCATGG TTGTTTTGTC TCACGTGATA ATTCAGAAAA TCCTTGCTCG 3654
   TTCCGCAAAT CCTAAAGCTC CTTTTGGAGA TGATATAGGA TGTGAAATAC AGAAACCTCA 3714
   GTGAAATCAA GAAATAATGA TCCCAGCCAG ACTGAGAAAA TGTAAGCAGA CAGTGCCACA 3774
   GTTAGCTCAT ACAGTGCCTT TGAGCAAGTT AGGAAAAGAT GCCCCCACTG GGCAGACACA 3834
   GCCCTATGGG TCATGGTTTG ACAAACAGAG TGAGAGACCA TATTTTAGCC CCACTCACCC 3894
   TCTTNGGTGC ACGACCTGTA CAGCCAAACA CAGCATCCAA TATGAATACC CATCCCCTGA 3954
   CCGCATCCCC AGTAGTCAGA TTATAGAATC TGCACCAAGA TGTTTAGCTT TATACCTTGG 4014
   CCACAGAGAG GGATGAACTG TCATCCAGAC CATGTGTCAG GAAAATTGTG AACGTAGATG 4074
   AGGTACATAC ACTGCCGCTT CTCAAATCCC CAGAGCCTTT AGGAACAGGA GAGTAGACTA 4134
   GGATTCCTTC TCTTAAAAAG GTACATATAT ATGGAAAAAA ATCATATTGC CGTTCTTTAA 4194
   AAGGCAACTG CATGGTACAT TGTTGATTGT TATGACTGGT ACACTCTGGC CCAGCCAGAG 4254
   CTATAATTGT TTTTTAAATG TGTCTTGAAG AATGCACAGT GANAAGGGGA GTAGCTATTG 4314
   GGAACAGGGA ACTGTCCTAC ACTGCTATTG TTGCTACATG TATCGAGCCT TGATTGCTCC 4374
   TAGTTATATA CAGGGTCTAT CTTGCTTCCT ACCTACATCT GCTTGAGCAG TGCCTCAAGT 4434
   ACATCCTTAT TAGGAACATT TCAAACCCCT TTTAGTTAAG TCTTTCACTA AGGTTCTCTT 4494
   GCATATATTT CAAGTGAATG TTGGATCTCA GACTAACCAT AGTAATAATA CACATTTCTG 4554
   TGAGTGCTGA CTTGTCTTTG CAATATTTCT TTTCTGATTT ATTTAATTTT CTTGTATTTA 4614
   TATGTTAAAA TCAAAAATGT TAAAATCAAT GAAATAAATT TGCAGTTAAG A 4665
(2) INFORMATION ZU SEQ ID NO: 2:
   (i) SEQUENZ CHARAKTERISTIKA:
      (A) LÄNGE: 1038 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Ser Gln Pro Glu Asp Ala Ser Gly Arg Cys Ala Gln Arg Phe Ser Thr
   1               5                   10                  15
   Leu Phe Ser Glu Leu Ala Val Arg Met Val Phe Ser Val Arg Gln Cys
               20                  25                  30
   Gly His Val Gly Arg Thr Glu Glu Val Leu Leu Thr Phe Lys Ile Phe
           35                  40                  45
   Leu Val Ile Ile Cys Leu His Val Val Leu Val Thr Ser Leu Glu Glu
       50                  55                  60
   Asp Thr Asp Asn Ser Ser Leu Ser Pro Pro Pro Ala Lys Leu Ser Val
   65                  70                  75                  80
   Val Ser Phe Ala Pro Ser Ser Asn Glu Val Glu Thr Thr Ser Leu Asn
                   85                  90                  95
   Asp Val Thr Leu Ser Leu Leu Pro Ser Asn Glu Thr Glu Lys Thr Lys
               100                 105                 110
   Ile Thr Ile Val Lys Thr Phe Asn Ala Ser Gly Val Lys Pro Gln Arg
           115                 120                 125
   Asn Ile Cys Asn Leu Ser Ser Ile Cys Asn Asp Ser Ala Phe Phe Arg
       130                 135                 140
   Gly Glu Ile Met Phe Gln Tyr Asp Lys Glu Ser Thr Val Pro Gln Asn
   145                 150                 155                 160
   Gln His Ile Thr Asn Gly Thr Leu Thr Gly Val Leu Ser Leu Ser Glu
                   165                 170                 175
   Leu Lys Arg Ser Glu Leu Asn Lys Thr Leu Gln Thr Leu Ser Glu Thr
               180                 185                 190
   Tyr Phe Ile Met Cys Ala Thr Ala Glu Ala Gln Ser Thr Leu Asn Cys
           195                 200                 205
   Thr Phe Thr Ile Lys Leu Asn Asn Thr Met Asn Ala Cys Ala Ala Ile
       210                 215                 220
   Ala Ala Leu Glu Arg Val Lys Ile Arg Pro Met Glu His Cys Cys Cys
   225 230 235 240
   Ser Val Arg Ile Pro Cys Pro Ser Ser Pro Glu Glu Leu Gly Lys Leu
   245 250 255
   Gln Cys Asp Leu Gln Asp Pro Ile Val Cys Leu Ala Asp His Pro Arg
   260 265 270
   Gly Pro Pro Phe Ser Ser Ser Gln Ser Ile Pro Val Val Pro Arg Ala
   275 280 285
   Thr Val Leu Ser Gln Val Pro Lys Ala Thr Ser Phe Ala Glu Pro Pro
   290 295 300
   Asp Tyr Ser Pro Val Thr His Asn Val Pro Ser Pro Ile Gly Glu Ile
   305 310 315 320
   Gln Pro Leu Ser Pro Gln Pro Ser Ala Pro Ile Ala Ser Ser Pro Ala
   325 330 335
   Ile Asp Met Pro Pro Gln Ser Glu Thr Ile Ser Ser Pro Met Pro Gln
   340 345 350
   Thr His Val Ser Gly Thr Pro Pro Pro Val Lys Ala Ser Phe Ser Ser
   355 360 365
   Pro Thr Val Ser Ala Pro Ala Asn Val Asn Thr Thr Ser Ala Pro Pro
   370 375 380
   Val Gln Thr Asp Ile Val Asn Thr Ser Ser Ile Ser Asp Leu Glu Asn
   385 390 395 400
   Gln Val Leu Gln Met Glu Lys Ala Leu Ser Leu Gly Ser Leu Glu Pro
   405 410 415
   Asn Leu Ala Gly Glu Met Ile Asn Gln Val Ser Arg Leu Leu His Ser
   420 425 430
   Pro Pro Asp Met Leu Ala Pro Leu Ala Gln Arg Leu Leu Lys Val Val
   435 440 445
   Asp Asp Ile Gly Leu Gln Leu Asn Phe Ser Asn Thr Thr Ile Ser Leu
   450 455 460
   Thr Ser Pro Ser Leu Ala Leu Ala Val Ile Arg Val Asn Ala Ser Ser
   465 470 475 480
   Phe Asn Thr Thr Thr Phe Val Ala Gln Asp Pro Ala Asn Leu Gln Val
   485 490 495
   Ser Leu Glu Thr Gln Ala Pro Glu Asn Ser Ile Gly Thr Ile Thr Leu
   500 505 510
   Pro Ser Ser Leu Met Asn Asn Leu Pro Ala His Asp Met Glu Leu Ala
   515 520 525
   Ser Arg Val Gln Phe Asn Phe Phe Glu Thr Pro Ala Leu Phe Gln Asp
   530 535 540
   Pro Ser Leu Glu Asn Leu Ser Leu Ile Ser Tyr Val Ile Ser Ser Ser
   545 550 555 560
   Val Ala Asn Leu Thr Val Arg Asn Leu Thr Arg Asn Val Thr Val Thr
   565 570 575
   Leu Lys His Ile Asn Pro Ser Gln Asp Glu Leu Thr Val Arg Cys Val
   580 585 590
   Phe Trp Asp Leu Gly Arg Asn Gly Gly Arg Gly Gly Trp Ser Asp Asn
   595 600 605
   Gly Cys Ser Val Lys Asp Arg Arg Leu Asn Glu Thr Ile Cys Thr Cys
   610 615 620
   Ser His Leu Thr Ser Phe Gly Val Leu Leu Asp Leu Ser Arg Thr Ser
   625 630 635 640
   Val Leu Pro Ala Gln Met Met Ala Leu Thr Phe Ile Thr Tyr Ile Gly
   645 650 655
   Cys Gly Leu Ser Ser Ile Phe Leu Ser Val Thr Leu Val Thr Tyr Ile
   660 665 670
   Ala Phe Glu Lys Ile Arg Arg Asp Tyr Pro Ser Lys Ile Leu Ile Gln
   675 680 685
   Leu Cys Ala Ala Leu Leu Leu Leu Asn Leu Val Phe Leu Leu Asp Ser
   690 695 700
   Trp Ile Ala Leu Tyr Lys Met Gln Gly Leu Cys Ile Ser Val Ala Val
   705 710 715 720
   Phe Leu His Tyr Phe Leu Leu Val Ser Phe Thr Trp Met Gly Leu Glu
   725 730 735
   Ala Phe His Met Tyr Leu Ala Leu Val Lys Val Phe Asn Thr Tyr Ile
   740 745 750
   Arg Lys Tyr Ile Leu Lys Phe Cys Ile Val Gly Trp Gly Val Pro Ala
   755 760 765
   Val Val Val Thr Ile Ile Leu Thr Ile Ser Pro Asp Asn Tyr Gly Leu
   770 775 780
   Gly Ser Tyr Gly Lys Phe Pro Asn Gly Ser Pro Asp Asp Phe Cys Trp
   785 790 795 800
   Ile Asn Asn Asn Ala Val Phe Tyr Ile Thr Val Val Gly Tyr Phe Cys
   805 810 815
   Val Ile Phe Leu Leu Asn Val Ser Met Phe Ile Val Val Leu Val Gln
   820 825 830
   Leu Cys Arg Ile Lys Lys Lys Lys Gln Leu Gly Ala Gln Arg Lys Thr
   835 840 845
   Ser Ile Gln Asp Leu Arg Ser Ile Ala Gly Leu Thr Phe Leu Leu Gly
   850 855 860
   Ile Thr Trp Gly Phe Ala Phe Phe Ala Trp Gly Pro Val Asn Val Thr
   865 870 875 880
   Phe Met Tyr Leu Phe Ala Ile Phe Asn Thr Leu Gln Gly Phe Phe Ile
   885 890 895
   Phe Ile Phe Tyr Cys Val Ala Lys Glu Asn Val Arg Lys Gln Trp Arg
   900 905 910
   Arg Tyr Leu Cys Cys Gly Lys Leu Arg Leu Ala Glu Asn Ser Asp Trp
   915 920 925
   Ser Lys Thr Ala Thr Asn Gly Leu Lys Lys Gln Thr Val Asn Gln Gly
   930 935 940
   Val Ser Ser Ser Ser Asn Ser Leu Gln Ser Ser Ser Asn Ser Thr Asn
   945 950 955 960
   Ser Thr Thr Leu Leu Val Asn Asn Asp Cys Ser Val His Ala Ser Gly
   965 970 975
   Asn Gly Asn Ala Ser Thr Glu Arg Asn Gly Val Ser Phe Ser Val Gln
   980 985 990
   Asn Gly Asp Val Cys Leu His Asp Phe Thr Gly Lys Gln His Met Phe
   995 1000 1005
   Asn Glu Lys Glu Asp Ser Cys Asn Gly Lys Gly Arg Met Ala Leu Arg
   1010 1015 1020
   Arg Thr Ser Lys Arg Gly Ser Leu His Phe Ile Glu Gln Met
   1025 1030 1035

## Beschreibung

Die Erfindung betrifft ein neues, für den Nebenhoden von Säugern spezifisches transmembranes Rezeptorprotein, für das Protein kodierende DNA-Sequenzen und spezifische Liganden, sowie deren Verwendung zur Herstellung von Mitteln zur Diagnose männlicher Infertilität und zur Steuerung der Spermienreifung.

Derzeit sind etwa 15% aller Paare in der Bundesrepublik Deutschland ungewollt kinderlos und ihr Anteil nimmt ständig zu. Die Ursachen für die Unfruchtbarkeit liegen zu gleichen Teilen bei Mann und Frau. Während jedoch die Ursachen bei der Frau weitgehend erforscht und diagnostizierbar sind, können beim Mann nur in ca. 30% der Fälle organische Ursachen festgestellt werden. Etwa ein Drittel der verbleibenden 70% lassen sich auf Oligospermie unbekannten Ursprungs zurückführen, während die Ursachen bei den restlichen Fällen nach dem bisherigen Wissensstand noch unklar sind. Insbesondere bei der idiopathischen Infertilität, bei der trotz einer ausreichenden Anzahl von Spermien im Ejakulat keine Befruchtung der weiblichen Eizelle erfolgt (H.W.G. Baker et al. (1986) Relative incidence of etiological disorders in male infertility. In: *Male Reproductive Dysfunction* (Hrsg. R.J. Santen und R.S. Swerdloff), S. 341 - 372. Marcel Dekker Inc., New York), wird eine Störung der im Nebenhoden (Epididymis) stattfindenden Spermienreifung vermutet.

Bei der Reifung der Spermien nimmt der Nebenhoden eine Schlüsselrolle ein. Er besteht beim Menschen aus einem einzigen etwa 5 m langen Kanal, der mäanderartig aufgewunden ist. Die im Hoden gebildeten, noch unreifen Spermien werden zum Nebenhoden transportiert und während einer 2 bis 3 Tage dauernden Passage einem Reifungsprozeß unterzogen. Das besondere Milieu des Nebenhodens sorgt dafür, daß die dort eingelagerten Spermien lange vital bleiben und überdies die besonderen physikalischen, immunologischen sowie biochemischen Eigenschaften befruchtungsfähiger Spermien erlangen.

Es ist demgemäß davon auszugehen, daß männliche Unfruchtbarkeit und insbesondere die idiopathische Infertilität in zahlreichen Fällen auf Störungen der im Nebenhoden ablaufenden Reifungsprozesse beruhen.

Obwohl, die anatomische Feinstruktur des humanen Nebenhodens makroskopisch, mikroskopisch und elektronenmikroskopisch sehr gut beschrieben ist (vgl. A.F. Holstein: Morphologische Studien am Nebenhoden des Menschen, Zwanglose Abhandlungen aus dem Gebiet der normalen und pathologischen Anatomie, **20**, 1-91 (1969)), sind dessen Proteinprodukte mit wenigen Ausnahmen (vgl. beispielsweise Tezon et al., Immunochemical localization of secretory antigens in the human epididymis and their association with spermatozoa, Biology of Reproduction, **32**, 591-597 (1985)) nur wenig erforscht. Fast alle Kenntnisse über dieses Organ stammen aus Arbeiten an Ratten, Mäusen, Hamstern, Ebern, Bullen oder gelegentlich Affen, wobei die Tier-spezifischen Unterschiede bekanntermaßen groß sind.

Die an verschiedenen Tierarten gewonnenen Erkenntnisse zur Spermienreifung beim Durchtritt durch den Nebenhoden lassen sich wie folgt zusammenfassen (vgl. T.G. Cooper: The Epididymis, Sperm Maturation and Fertilisation, Springer Verlag, Berlin, (1986)):
(a) Entwicklung einer gerichteten Vorwärtsbewegung und Befähigung zur Hyperaktivierung der Spermien.
(b) Verhinderung einer vorzeitigen Kapazitation, d.h. der Bereitschaft, die Akrosomenreaktion durchzuführen, wobei Dekapazitationsfaktoren, bei denen es sich vermutlich um epididymale Polypeptide handelt, eine regelnde Rolle spielen.
(c) Veränderung der Oberflächenantigene der Spermien, um die Bindung zwischen Spermien und Eizelle zu begünstigen.
(d) Veränderung der Spermienmembran, um die Fusion mit dem Ei zu ermöglichen.

Das diese Prozesse auslösende Stoffwechselgeschehen im Nebenhoden ist selbst bei den untersuchten Tieren weitgehend ungeklärt. Es konnte jedoch anhand von Gelelektrophoresen gezeigt werden, daß im Nebenhoden einige Polypeptide zu der aus der Rete testis stammenden Seminalflüssigkeit hinzukommen. Unter diesen sind bei der Ratte die Polypeptide (a) bis (e) von Cooper (a.a.O., Tab. 20) vorhanden, in denen das sogenannte "acidic epididymal glycoprotein (AEG)° (Lea et al., (1978)) bzw. die Polypeptide B bis E von Brooks und Mitarbeitem (D.E. Brooks und J. Higgins, Characterization and androgen-dependence of proteins associated with luminal fluid and spermatozoa in the rat epididymis, Journal of Reproduction and Fertility, **59**, 363-375, (1980)) enthalten sind. Die Ergebnisse dieser Modelle weisen darauf hin, daß die Bildung dieser Polypeptide unter Androgeneinwirkung steht.

Die aus Tierversuchen gewonnen Erkenntnisse lassen sich jedoch aufgrund der hohen Gewebe- und Spezies-Spezifität nicht auf den Menschen übertragen. Beispielsweise wurden die bei der Ratte wesentlichen epididymalen Polypeptide oder die sie kodierenden mRNA's in keinem anderen Gewebe und, abgesehen von der Maus, in keiner anderen Spezies gefunden (D.E. Brooks et al., Europ. J. Biochem., **161**, 13-18 (1986); J. Biolog. Chem., **261,** 4956-4981. (1986)).

Aus der EP-A-0 440 321 sind 5 spezifische Polypeptide des menschlichen Nebenhodens sowie für diese Polypeptide kodierende Nukleotidsequenzen bekannt, die in engem Zusammenhang mit der Spermienreifung im Nebenhoden stehen. Diese Nukleotidsequenzen sowie die entsprechenden Expressionsprodukte als auch dagegen gerichtete Antikörper sind geeignet zur Diagnose und zur Verwendung bei der Behandlung männlicher Infertilität.

Die der vorliegenden Erfindung zugrundeliegende Aufgabe besteht daher in der Bereitstellung weiterer Mittel, mit deren Hille Störungen des Proteinstoffwechsels im Nebenhoden des Menschen-und insbesondere männliche Infertilität - diagnostiziert und gegebenenfalls therapiert werden können.

Zur Lösung der Aufgabe werden das erfindungsgemäße, Nebenhodenspezifische Rezeptorprotein (NSRP) gemäß SEQ ID NO 2, sowie für das erfindungsgemäße NSRP kodierende DNA-Sequenzen, insbesondere solche gemäß SEQ ID NO 1 und von dieser abgeleitete DNA-Sequenzen, sowie unter Berücksichtigung der Degeneration des genetischen Codes mit diesen DNA-Sequenzen übetelnatimmende DNA-Sequonzen vorgeschlagen.

Die Erfindung schließt ferner homologe Aminosäure- und entsprechende DNA-Sequenzen mit einem Homoiogiegrad von mindestens 90% ein. Schließlich sind Nukteotidsequonzen, welche mit den zuvor genannten unter den nachfolgend definierten Bedingungen hybridisieren, erfindungsgemäß eingeschlossen und insbesondere als Sonden geeignet.

Die Isolierung und Identifizierung von Nebenhoden-spezifischen Ftezeptorprofe4nen, welche an der Schaffung des oben beschriebenen Nebenhodenmilieus beteiligt sind, nach konventionellen Verfahren ist wegen der geringen Mengen des zur Verfügung stehenden Gewebes nicht möglich.

Im Rahmen der vorliegenden Erfindung ist es nunmehr erstmals gelungen, ein spezifisches transmembranes Rezeptorprotein des Nebenhodens von Säugern nachzuweisen, zu charakterisieren und seine Herstellung mittels rekombinanter Verfahren oder chemischer Synthese zu ermöglichen.

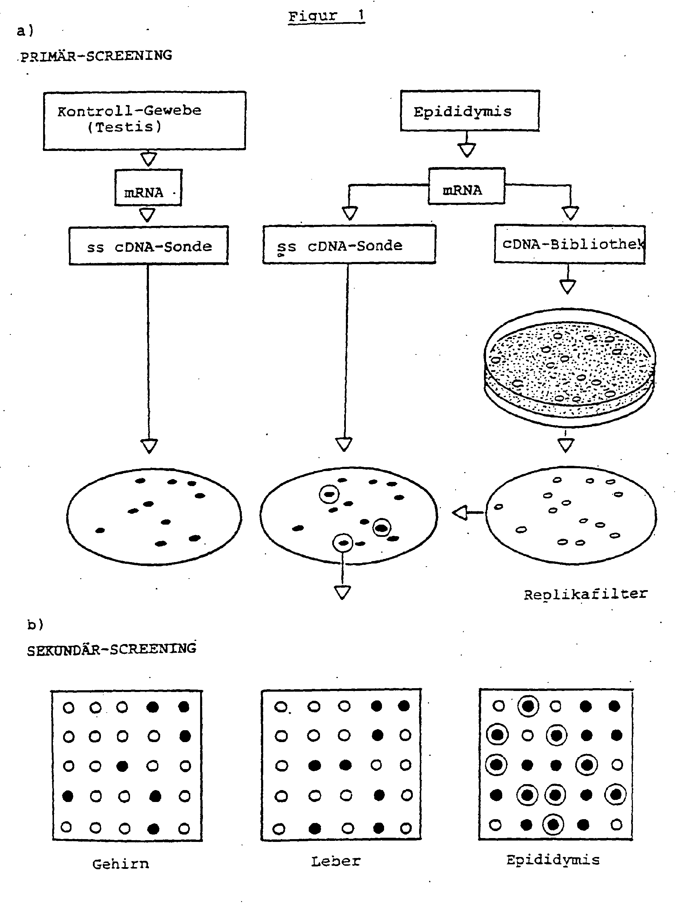

Zur Identifizierung des erfindungsgemäßen Rezeptorproteins wurde zunächst eine cDNA-Bibliothek aus epididymaler mRNA in Lambda gt11(Clontech, California) erstellt, sodann wurden die Nebenhoden-spezifischen Rekombinanten in einer Folge von differentiellen Hybridisierungsschritten selektiert (vgl. Figuren 1 u. 3 bis 5) und anschließend In dem bakteriellen Plasmid pBS (Stratagene, California, USA) subkloniert und charakterisiert.

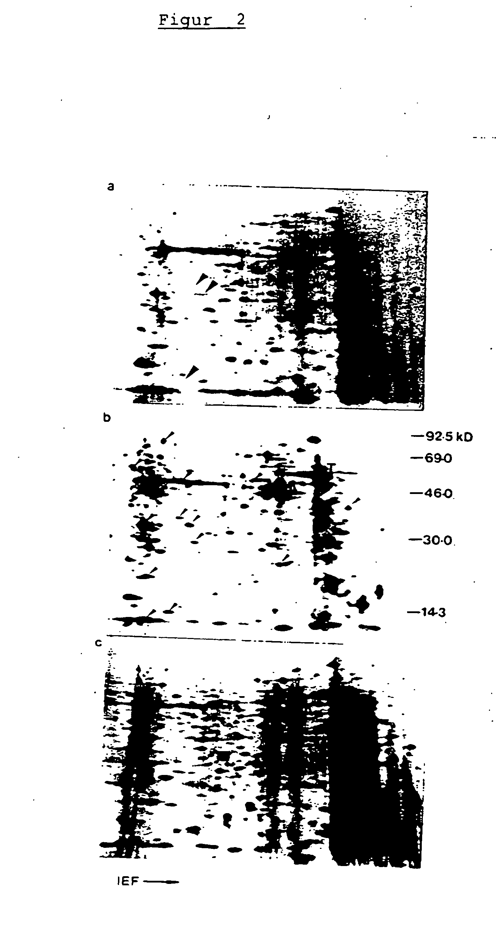

Das differentielle Screening zielte darauf ab, Klone von mRNA's zu isolieren, welche im Gewebe des Nebenhodens, nicht aber im Gewebe anderer und funktionsverwandter Organe vorhanden sind. Bei dem erfindungsgemäß eingesetzten Verfahren diente im ersten Schritt menschliches Hodengewebe als Vergleichsmaterial. Da einige der als Ausgangsmaterial verwendeten Gewebeproben von orchiektomierten Männern stammten, die mit verschiedenen Medikamenten behandelt worden waren, wurde zunächst überprüft, ob und in welchem Umfang die Medikamentation Einfluß auf das jeweilige Polypeptidmuster ausübt. Es konnte jedoch an 7 Patienten unterschiedlichen Alters und unterschiedlicher Medikamentation gezeigt werden (vgl. Beispiel 2 und Figur 2a bis c), daß die Muster durch vorherige Verabreichung von Medikamenten nur unwesentlich beeinflußt werden und daß auch die individuellen Abweichungen gering sind.

Wie in Figur 1 schematisch dargestellt ist, erfolgte das Screening erfindungsgemäß in einer primären und einer sekundären Stufe, wobei In der primären Stufe durch Kreuzhybridisieren mit Testis-mRNA potentiell Nebenhodenspezifische Klone isoliert wurden und diese in der sekundären Stufe durch Kreuzhybridisieren mit mRNA aus Gehirn und Leber weiter selektioniert wurden (vgl. Beispiel 3 und Figuren 3 und 4).

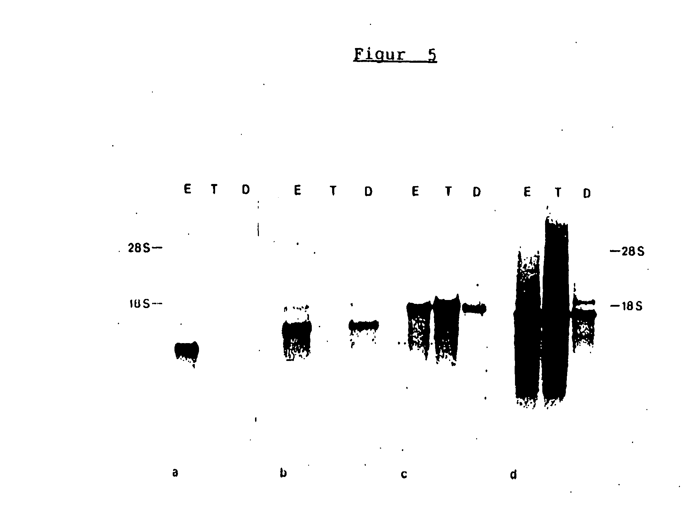

Durch nachfolgende Northern-Blot-Analyse gegen Gesami-mRNA aus Hoden und menschlicher Decidua konnte die Zahl der in Betracht kommenden Klone weiter eingeengt und schließlich sechs unabhängigen cDNA-Familien zugeordnet werden (vgl. Beispiel 3 und Figur 5). Aus einer dieser cDNA-Femilien wurde der für das erfindungsgemäße Rezeptorprotein kodierende cDNA-Klon gemäß SEQ ID NO 1 isoliert.

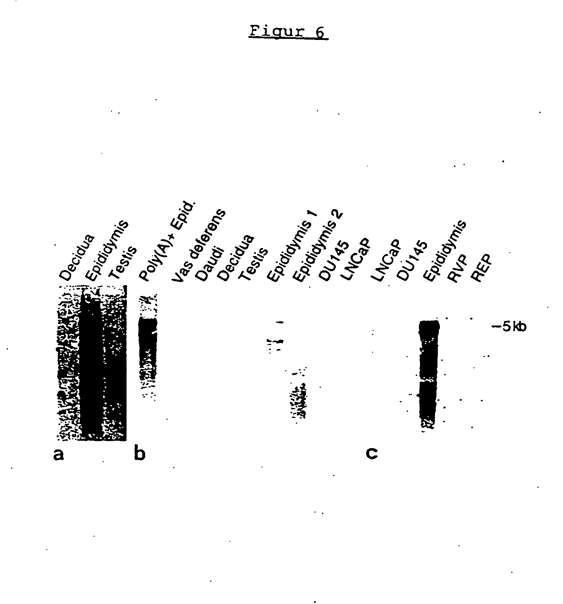

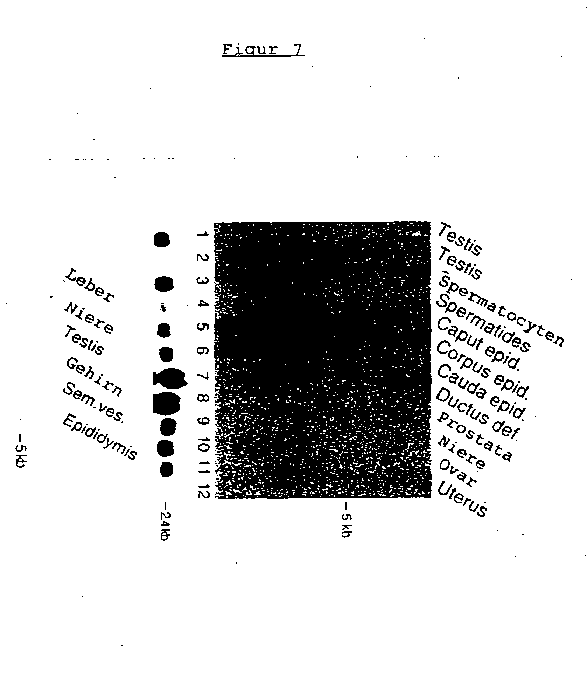

Die erfindungsgemäß gewonnenen Ergebnisse zeigen, daß die diesem Klon zugrunde liegende mRNA nicht in anderen menschlichen Geweben synthetisiert wird (vgl. Figur 6); es handelt sich demgemäß um ein Nebenhoden-spezifisches Molekül. Die Figuren 6 und 7 zeigen die jeweils zugrundeliegenden Autoradiogramme.

Die Nukleotid- sowie die entsprechende Aminosäuresequenz des erfindungsgemäßen cDNA-Kions wurden wie in Beispiel 7 angegeben analysiert. Das Ergebnis ist in SEQ ID NO 1 wiedergegeben.

Die Spezifität dieses Klons und damit des entsprechenden Expressionsprodukts ergibt sich aus seiner ausschließlichen Northern-Hybridisierung mit aus humanem Nebenhoden extrahierter RNA, die in keinem anderen untersuchten humanen Gewebe einschließlich den Organen Gehim und Leber durch Kreuzhybridisierung nachgewiesen werden konnte. Die Gewebespezifität wurde ferner dadurch bestätigt, daß eine homologe, unter Anwendung der PCR-Technologie hergestellte Gensonde aus der Ratte ebenfalls ausschließlich mit RNA aus dem Nebenhodengewebe hybridisierte, nicht aber mit RNA eines anderen Gewebes dieser Tierart (vgl. Figur 7).

Eine vollständige Sequenzierung einer Reihe von homologen cDNA-Klonen, die aus zwei humanen NebenhodencDNA-Bibliotheken isoliert bzw. durch Anwendung des 5'RACE-Verfahrens (M.A. Frohman, RACE: Rapid Amplification of cDNA ends, In: PCR Protocols: A Guide to Methode and Applications, Academic Press Inc., S. 28 - 38 (1990)) ermittelt wurden, ergab ein einheitliches Gentranskript gemäß SEQ ID NO 1 mit einer Länge von 4665 Nukleotiden, welche mit der Länge der mRNA korrespondiert, die durch Northern-Hybridisierung bestimmt wurde. Diese Sequenz umfaßt einen vollständigen Leserahmen, der für ein Polypeptid von 1038 Aminosäuren kodiert.

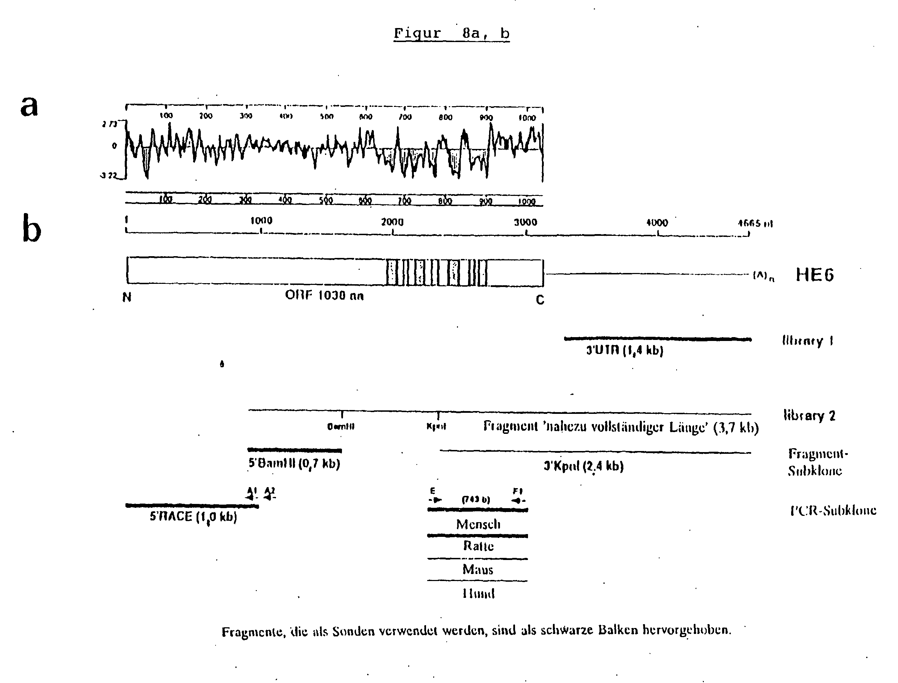

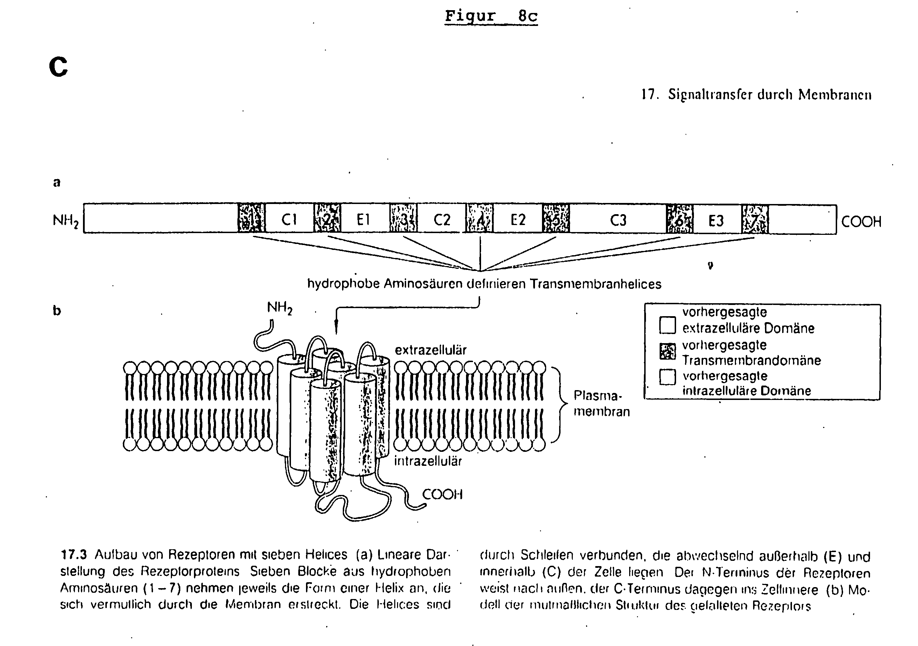

Die Homologie der erfindungsgemäßen Aminosäure- und DNA-Sequenzen (SEQ ID NO 1 und 2) mit den in verschiedenen internationalen Datenbanken (NIH-Genbank data-base, EMBL, PIR) gespeicherten Sequenzen wurde analysiert. Dabei zeigte es sich, daß die maximale gefundene Homologie gegenüber der "Sekretin/VIP Superfamilie" von G-Protein gekoppelten Rezeptoren (W.C. Probst et al., Sequence Alignment of G-Protein Coupled Receptor Superfamily, DNA and Cell Biology, 11, S. 1 - 20 (1992)) bestand und 25% betrug. Es handelt sich demgemäß um einen bisher unbekannten Rezeptor, dessen langes N-terminales Ende auf eine extrazelluläre Domäne hinweist, die mit derjenigen von Glykoprotein-Rezeptoren vergleichbar ist, d.h. um einen neuen Rezeptor für einen großen Glykoprotein-Liganden, der aus dem testikularen Sekret stammt. Anhand einer Hydrophobizitätsanalyse (Bsp. 7) wurden sieben klar definierte hydrophobe Bereiche aufgezeigt, welche als transmembrane Domänen zu bezeichnen sind (vgl. Figur 8a). Die Struktur des erfindungsgemäßen Proteins kennzeichnet dieses als membranständigen Rezeptor, welcher der Genfamilie der G-Protein gekoppelten Rezeptoren zuzuordnen ist (s.o.). Er besitzt die für diese Gruppe von Proteinen typische G-Protein-Bindungsdornäne, mit deren Hilfe Informationen durch den Rezeptor transduziert und über G-Proteine in das Innere von Zellen weitervermittelt werden (L. Bimbaumer, J. Abramowitz und A.M. Brown, Receptor-effector coupling by G proteins, Biochem. Biophys. Act. 1031, 163 - 224 (1990)).

Das erfindungsgemäße Rezeptorprotein liegt im Nebenhoden in hoher Abundanz vor (die mRNA macht etwa 0,01 % der cDNA-Bibliothek aus)-und *in situ* Hybridisierungsversuche lokalisierten die mRNA in den Epithelzellen, welche den Ductus epididymidis auskleiden.

Ferner handelt es sich um ein unter den Säugerspezies hochkonserviertes Molekül, da bei den bisher untersuchten Säugerspezies eine Homologie der Expressionsprodukte von etwa 90% bei gleicher Gewebespezifität gefunden wurde.

Die für das neue erfindungsgemäße Rezeptorprotein kodierenden Nukleotidsequenzen können nach herkömmlichen Verfahren über geeignete Vektoren in pro- oder eukaryontische Wirtszellen transferiert und dort als Protein exprimiert werden.

Erfindungsgemäß sind sämtliche DNA-Sequenzen geeignet und eingeschlossen, die nach Transformation geeigneter pro- und/oder eukaryontischer Wirtszellen die Gewinnung von Nukleinsäuren zur Verwendung als Diagnostika und/oder die Expression von Proteinen oder Polypeptiden gewährleisten. Diese Sequenzen in einzel- oder doppelsträngiger Form umfassen insbesondere:
a) Die im Sequenzprotokoll unter der SEQ ID NO 1 angegebene Nukleotidsequenz, die dort angegebene Sequenz von den Nukleotiden 1 bis 3114, zu den vorgenannten Sequenzen homologe Sequenzen mit einem Homologiegrad von mindestens 90% oder Komplementäre Sequenzen;
b) Nukleotidsequenzen, die mit dem proteinkodierenden Bereich der unter a) angegebenen Nukleotidsequenz hybridisieren, beispielsweise unter den in den Beispielen 3 oder 4 angegebenen Hybridisierungsbedingungen;
c) Nukleotidsequenzen, die mit Ausnahme der durch die Degeneration des genetischen Codes verursachten Abweichungen mit den unter a) und/oder b) genannten Sequenzen hybridisieren.

Als geeignete Vektoren für prokaryontische Wirtszellen dienen beispielsweise Plasmide der pET-Serie (Rosenburg et al., "Vectors for selective expression of cloned DNAs by T7 RNA Polymerase", Gene, 56, S. 125-135 (1987)), der pGEX-Serie (Pharmacia, Freiburg), der pRIT-Serie (Pharmacia, Freiburg), und der pH6EX3-Serie (Berthold et al., "Purification of Recombinant Antigenic Epitopes of the Human 68-kDa (U1) Ribonucleoprotein Antigen Using the Expression System pH6EX3 Followed by Metal Chelating Affinity Chromatography", Protein Expression and Purification, 3, S. 50-56 (1992)). Beispiele für prokaryontische Wirtszellen sind die üblichen Laborstämme von Escherichia coli K12 sowie die Stämme BL21 (DE3) und LE392.

Beispiele-geeigneter Vektorsysteme für eukaryontische Wirtszellen sind im Falle von Säuger-Zellen SV40 Viren, Polyoma-Viren, Adenoviren, verschiedene Retroviren, Papillomaviren (P.W.J. Rigby, "Expression of cloned genes in eucaryotic cells using vector systems derived from viral replicons", Genetic Engineering, Bd. 3, S. 84-141 (1982)), sowie Vaccinia-Viren (Mackett et al., "The construction and characterization of Vaccinia Virus recombinants expressing foreign genes", DNA Cloning, Bd. II, (1985), IRL Press, Oxford), und Derivate derselben sowie solche Plasmide, die Teile viraler Gene aufweisen (z.B. pSV2) und als "Shuttle-Vektoren" eingesetzt werden können (P.W.J. Rigby, a.a.O.). Als Vektoren für Insekten-Zellen kommen beispielsweise pJVETL-Bakuloviren (Invitrogen, San Diego, Calif., USA) in Betracht, während für Hefe-Zellen z.B. pJP31, YEp- und Ylp-Plasmide (Carter et al., "Expression and secretion of foreign genes in Yeast", DNA Cloning, Bd. III, (1987), IRL Press, Oxford) verwendet werden können.

Geeignete Säuger-Zellen sind z.B. COS-Zellen, CHO-Zellen, AtT20-Zellen und N1H3T3-Zellen (Rigby, a.a.O., Mackett et al., a.a.O.), während geeignete Insekten-Zellen z.B. Sf9, Sf21 und TN5 (Invitrogen, San Diego, Calif., USA) sind. Geeignete Hefe-Zellen sind z.B. X4003-5B (Carter, a.a.O.).

Ferner kann das erfindungsgemäße Rezeptorprotein nach bekannten Verfahren chemisch synthetisiert werden (J.M. Stewart, "Synthesis and use of neuropeptides", Neuroendocrine Peptide Methodology, ed. P.M. Conn, Academic Press, New York, S. 815-844 (1989)). Das gleiche gilt für Polypeptide oder Peptidepitope mit gleicher Immunogenität, die von Fragmenten oder syngenen Sequenzen der erfindungsgemäßen DNA-Sequenz (SEQ ID NO 1) kodiert sind.

Die erfindungsgemäßen Polypeptide bzw Proteine, die für diese kodierenden Nukleotidsequenzen einschließlich ihrer komplementären Sequenzen, sowie auf Basis der Polypeptide und Proteine hergestellte Antikörper bieten erstmals die Möglichkeit, Störungen im Proteinstoffwechsel des Nebenhodenepithels zu diagnostizieren und gegebenenfalls zu therapieren bzw. neue kontrazeptive Mittel bereitzustellen.

So können beispielsweise die oben genannten Nukleotidsequenzen mit Markem versehen und als Sonden zur in situ-Hybridisierung bei der Gewebediagnostik von Biopsieproben oder Dünnschnitten verwendet werden, um den physiologischen Zustand des Gewebes hinsichtlich der Präsenz und Konzentration der offindungsgemäßen Rezeptorproteine zu bestimmen und mit Standard-Werten zu vergleichen.

Polyklonale und monoklonale Antikörper zur Verwendung in immunologischen Nachweisverfahren können mit Hilfe des erfindungsgemäßen hochreinen Polypeptids auf bekannte Weise hergestellt werden. Derartige Antikörper lassen sich auf der Basis des vollständigen Rezeptorproteins herstellen (vgl. Beispiele 8, 9).

Die Antikörper können markiert sein und dem Nachweis des erfindungsgemäßen Rezeptorproteines in vitro oder in vivo dienen.

Das erfindungsgemäße Rezeptorprotein kann ferner in markierter oder unmarkierter Form als Antigene zur Identifizierung von Autoantikörpem in den Seren infertiler Männer dienen. Diese Möglichkeit ist von besonderer Bedeutung, da vermutet wird, daß die Infertilität in einem großen Teil der Fälle auf das Vorhandensein von Autoantikörpern gegen wesentliche Komponenten des Fortpflanzungssystems zurückzuführen ist. Die bisher zur Verfügung stehenden Testmethoden messen jedoch nur Antikörper, die gegen einige Sperm-Oberflächen-Antigene gerichtet sind, wobei ein ausreichend hoher Titer der Antikörper vorhanden sein muß, um die Sperm-Agglutination erfolgen zu lassen. Es wird jedoch angenommen, daß Antikörper in weitaus niedrigeren Titern vorhanden sein und Infertilität verursachen können. Diese können mit dem erfindungsgemäß hergestellten reinen Rezeptorprotein als Antigen erfaßt werden.

Ausgehend von der erfindungsgemäß offenbarten Aminosäuresequenz bieten sich hinsichtlich der Gewinnung von Antigenen zur Herstellung von Antikörpern zwei unterschiedliche Verfahren an.

Zum einen läßt sich mit Hilfe eines Computers eine potentiell immunogene Region der interessierenden Proteinsequenz auswählen, welche einerseits relativ hydrophil ist und damit an der Außenseite des Proteinmoleküls liegt, andererseits aber nicht durch Bildung von Cystein-Doppelbrücken oder möglichen Glykosylierungsstellen in ihrer sterischen Konformation gestört wird.

Dieser Peptidbereich wird anschließend gegebenenfalls zusammen mit flankierenden Aminosäuren synthetisiert, nachfolgend an Trägersubstanzen gekoppelt und als Immunogen für die Induktion von Antikörpern eingesetzt (vgl. Beispiel 9).

Alternativ läßt sich beispielsweise die für das interessierende Polypeptid kodierende Nukleotidsequenz in einen geeigneten Expressions-Plasmidvektor umklonieren. Nach anschließender Transformation in geeignete Bakterien erlauben diese Vektoren eine induzierbare Expression des kodierten Polypeptids. Das auf diese Weise hergestellte bakteriogene Protein kann nach Aufreinigung aus dem Bakterienextrakt direkt als Antigen für eine Immunisierung zur Induktion von Antikörpern verwendet werden (vgl. Beispiel 8).

Die Antikörper können mit einem nachweisbaren Marker wie z.B. einem fluoreszierenden Molekül (Fluorophor) versehen und beispielsweise bei Gewebeproben eingesetzt werden, um das Vorhandensein des Nebenhoden-spezifischen Rezeptorproteins im Nebenhodenepithel mit Hilfe der Immunfluoreszenz zu bestimmen.

Die Identifizierung und Charakterisierung des erfindungsgemäßen Rezeptorproteins als hochspezifisches Vermittler-Molekül, welches ausschließlich auf den Zellen des Nebenhodenepithels von Säugern vorhanden und in der Lage ist, Informationen zur Steuerung der Zellfunktion innerhalb der Zellen des Epithels weiterzuleiten, macht es zu einem äußerst interessanten Kandidaten zur Diagnose und ggfs. zur Beeinflussung der zuvor erwähnten Physiologie des Nebenhodenepithels. Durch eine derartige gezielte positive Beeinflussung kann der Spermienreifungsprozess im Nebenhoden auf therapeutischem Wege verbessert werden, indem beispielsweise der im zu behandelnden Individuum fehlende oder in unzureichender Menge gebildete Ligand medikamentös verabreicht wird. Andererseits kann eine negative Beeinflussung des Epithels zu einer Verschlechterung der Spermienreifung und damit zur Bereitstellung neuer kontrazeptiver Mittel für den Mann führen. Zu diesem Zwecke ist beispielsweise ein künstlicher Ligand geeignet, der an das erfindungsgemäße Rezeptorprotein zwar fest bindet, jedoch keine Signalübertragung bzw weiterleitung auslöst. Ferner können gegen das erfindungsgemäße Rezeptorprotein gerichtete Antikörper eingesetzt werden, um den oder die für das Rezeptorprotein spezifischen Liganden im Wege einer kompetitiven Verdrängung an der Bindung und somit an einer Signalauslösung zu hindern.

Durch Anwendung bestimmter Verfahren wie z.B. Phagen-Display und Peptid-Display (J.K. Scott und G.P. Smith, Searching for peptide ligand with an epitope library, Science 249, S. 386-390 (1990); J.J. Devlin et al., Random peptide libraries: a source of specific protein binding molecules, Science 249, S. 404-406 (1990)), Evolutive Biotechnologie (M. Eigen, Selforganization of matter and the evolution of biological macromolecules, Die Naturwissenschaften 58, S. 465-523 (1971); M. Eigen, Automated molecular evolution, Max-Planck-Institut f. Biophysikalische Chemie (1991)) ist es dem Fachmann ohne weiteres möglich, künstliche Liganden zu identifizieren, die die Fähigkeit besitzen; spezifisch und mit hoher Affinität an ein solches Rezeptorprotein zu binden und entweder agonistisch oder antagonistisch auf dessen Fähigkeit zur Signalübertragung zu wirken (Bsp. 11). Auf diesem Wege können Moleküle bereitgestellt werden, mittels derer die Zellen, welche das Rezeptorprotein exprimieren, in ihrer Physiologie positiv (therapeutisch) oder negativ (kontrazeptiv) beeinflußbar sind.

Die Erfindung wird nachfolgend anhand von Beispielen im einzelnen erläutert.

### Beispiel 1

### RNA-Präparation

Gesamt-RNA wurde nach dem Verfahren von J.M. Chirgwin et al., Isolation of biologically active ribonucleic acid from sources enriched in ribonuclease, Biochemistry, **18**, 5294, (1979), aus den gefrorenen Geweben menschlicher Hoden (Testis) und Nebenhoden (Epididymis), die Männern mit Prostatakarzinom im After von 58 und 74 Jahren durch Orchiektomie entnommen worden waren, unter Einsatz von Guanidin-Isothiocyanat extrahiert und anschließend durch Cäsiumehlorid-Dichtegradienten-Zentrifugation gereinigt. Durch Verwendung einer Oligo(dT)-Collulosesäule konnte Poly(A)+RNA affinitätschromatographisch angereichert werden, wie von H. Aviv & P. Leder, Purification of biologically active globin messenger RNA by chromatography on oligothymidylic acid-cellulose, Proc. Natl. Acad. Sci., USA, **69**, 1408-1412, (1972), beschrieben. Die RNA-Proben wurden mit Ethanol gefällt und nach Resuspendieren in sterilem Wasser in einer Konzentration von 1 µg/µl bei -80°C aufbewahrt.

### Beispiel 2

### In vitro-Translation und Charakterisierung der Produkte durch zweidimensionale Gelelektrophorese

0,5 bis 1 µg der Poly(A)+RNA von Nebenhoden und Hoden aus Beispiel 1 wurden jeweils in einem zellfreien System eines Retikulozyten-Lysats aus Kaninchen (New England Nuclear (NEN), Dreieich, BRD) in vitro-translatiert. Die Synthese wurde in Gegenwart von (³⁵S)-Methionin (spezifische Aktivität > 1000 Ci/mmol) durchgeführt. Die resultierenden Proteinprodukte wurden anschließend in einem zweidimensionalen Gel elektrophoretisch aufgetrennt (vgl. P.H. O'Farrel, High resolution two-dimensional electrophoresis of proteins, J.Biol. Chem., **250,** 4007-4021 (1975)). Für die erste Dimension wurde jeweils eine Menge von 350 000 cpm der mit (³⁵S)-Methionin markierten Translationsansätze auf das Gel aufgetragen und bei einer angelegten Spannung von 10 000 V.h isoelektrisch fokussiert. Der pH-Gradient lag zwischen 4,0 und 7,5. Die Auftrennung der Polypeptide nach ihrem Molekulargewicht wurde in der zweiten Dimension innerhalb eines linearen Gradienten von 7,5 - 15% Acrylamid durchgeführt (vgl. D.M. Neville & H. Glossmann, Molecular weight determination of membrane protein and glycoprotein subunits by discontinous gel electrophoresis in dodecylsulfate, Meth, Enzym., **32**, 92-102, (1974)). Zur Abschätzung der Molekulargewichte wurde in der zweiten Dimension ein mit ¹⁴C markierter Protein-Marker (Amersham, GB) aufgetragen. Anschließend wurde das Gel mit Hilfe des autoradiographischen Films Kodak X-AR5 fluorographiert (vgL W.M. Bonner & R.A. Laskey, A film detection method for Tritium-labelled proteine and nucleic acids in polyacrylamide gels, Europ. J. Biochem., **46**, 83-88, (1974)). Die Expositionszeit betrug 8 Tage. Die Ergebnisse sind in Figur 2 wiedergegeben.

Die die Ergebnisse darstellende Figur 2 zeigt das Muster der zellfrei synthetisierten Translationsprodukte, die sich von Nebenhoden- bzw. Hoden-spezifischer Poly(A)+RNA ableiten.

Insbesondere zeigt Figur
2a: Nebenhodengewebe eines 74 Jahre alten, mit Cyproteronacetat behandelten Orchlektomie-Patienten.
2b: Nebenhodengewebe eines 58 Jahre alten, nicht mit Medikamenten behandelten Orchiektomie-Patienten,
2c: Hodengewebe desselben, nicht mit Medikamenten behandelten Patienten (siehe Figur 2b).

Nebenhoden-spezifische Translationsprodukte, deren korrespondierende Banden In Figur 2c (Translationsprodukte von mRNA aus Hoden) nicht auftauchen, sind durch kleine Pfeile gekennzeichnet (siehe Figur 2b).

Eine Veränderung der expression Im Nebenhoderigewebe durch vorherige Behandlung mit Antiandrogenen zeigt ein Vergleich der Figuren 2a und 2b. Banden, die in Figur 2a gegenüber Figur 2b reduziert sind, wurden durch große Pfeile markiert. Die Molekulargewichte des Proteinmarkers sind in Kilodalton angegeben. Actin(A)- und Tubulin(T)-ähnliche Produkte sind jeweils gekennzeichnet.

### Beispiel 3

### Erstellung der cDNA-Gesamtbibliothek und Auswahl potentiell spezifischer Klone

Die Kultivierung, Handhabung und Erhaltung von Bakterien und Bakteriophagen sowie der Einsatz DNA-rekombinierender Techniken erfolgte nach den Angaben von T. Maniatis et al., Molecular Cloning, A Laboratory Manual, Cold Spring Harbour Laboratory, Cold Spring Harbour, N.Y., (1982).
a) Für die Erstellung der cDNA-Bibliothek wurde Poly(A)⁺RNA aus dem Nebenhoden-Gewebe eines nicht medikamentös behandelten Patienten eingesetzt (bezüglich Muster der In vitro-Translationsprodukte, vgl. Figur 2b). 20 µg von Poly(A)⁺RNA gemäß Beispiel 1 wurden unter Verwendung von Oligo(dT) als Primer revers transkribiert (vgl. U. Gubler & B.J. Hoffmann, A simple and very efficient method for generating cDNA libraries, Gene, 15, 263-269. (1983)).
   Die doppelsträngigen cDNA-Konstrukte wurden auf beiden Seiten mit EcoRI-Linkern ligiert und anschließend in die EcoRI-Klonierungsstelle des Bakteriophagen Lambda gt11(Clontech California) eingeführt.
   Zum Auffinden von Klonen vollständiger Länge wurde eine weitere. Nebenhoden-spezifische cDNA-Bibliothek im wesentlichen nach dem dargelegten Verfahren erstellt, jedoch mit der Abweichung, daß die cDNA-Konstrukte unidirektionell kloniert wurden. Dies erfolgte, indem das jeweilige 5'-Ende mit einem EcoRI-Linker und das jeweilige 3'-Ende mit einem Xhol-Linker ligiert und das jeweilige Konstrukt sodann in den Bacteriophagen Lambda Uni-ZAP (Stratagene, La Jolla, Kalifornien) eingeführt.
   Bakterien der E. coli-Stämme Y 1090 und XL1-Blue wurden bis zur logarithmischen Phase angezogen, in Weich-Agarose überführt und zusammen mit der nicht amplifizierten Lambda-Bibliothek in einer Phagandichte von 500 bis 1000 pfu pro Petrischale (15 cm) ausplattiert und 8 Stunden lang bei 42°C bzw. 37°C inkubiert.
   Für das differentielle Screening wurden die Plaques einer jeden Schale nachfolgend auf zwei Nitrocellulose-Filter (Schleicher & Schüll, Darmstadt, BRD, BA85) Replika-plattiert, wobei die Inkubationszeit für das erste Filter 1 Minute, für das zweite Filter 2 Minuten betrug.
   In dieser Weise wurden Replika-Flilter von insgesamt 20 Petrischalen hergestellt und anschließend mit jeweils zwei einzeisträngigen, radioaktiv markierten cDNA-Sonden (vgl. b, unten) (positiv/negativ) hybridisiert.
b) Die Sonden aus Poly(A)+RNA von Nebenhoden (liefert positive Sonde) und aus Poly(A)+RNA von Hoden (liefert negative Sonde) wurden unter Verwendung von Oligo(dT) als Primer wie folgt hergestellt:
   Beide RNA-Spezies entstammten den entsprechenden Geweben eines Patienten, der keiner medikamentösen Vorbehandlung ausgesetzt worden war. Jeweils 1 µg der Poly(A)+RNA gemäß Beispiel 1 wurde 5 Minuten lang bei 65°C in einem Volumen von 2 µl denaturiert Nach schnellem Abkühlen der Ansätze auf Eis wurden jeweils folgende Bestandteile nacheinander zugegeben:
   - 2 µl Oligo(dT) (100 µg/ml)
   - 1 µl dNTP-Mix (dATP, dGTP, dTTP jeweils 2 mM)
   - 1 µl 40 mM Natriumpyrophosphat
   - 1 µl RNasin (Amersham; GB)
   - 2 µl 10x Reverse Transkriptase-Puffer.(500mM Tris-CI (pH8,5), 500 mM KCI, 100 mM MgCl₂, 100 µg/ml BSA, 10 mM EDTA, 10 mM Dithiotreitol)
   - 20 Einheiten AMV reverse Transkriptase (Boehringer Mannheim, BRD)
   - 10 µl (α-³²P)-dCTP (NEN, 10 µ Ci/µl; S.A. > 3000 Ci/mmol).

Die Reaktionsansätze wurden 15 Minuten lang bei 42°C inkubiert. Nach Zugabe von 1 µl "Chasemix" (enthaltend alle vier dNTP's in einer Konzentration von jeweils 10 mM) wurde die Inkubation für weitere 20 Minuten fortgesetzt. Die Reaktion wurde durch Zugabe von jeweils 1 µl 0,5 M EDTA abgestoppt und die Produkte ohne weitere Reinigung mit Ethanol gefällt. Die mit einer spezifischen Aktivität von > 10⁸ cpm/µg markierten Sonden wurden mit den oben genannten Replika-Filtern parallel hybridisiert. Die Hybridisierung wurde in 5x Denhardt's-Lösung, 4x SET (200 mM Tris (pH 8,0), 20 mM EDTA, 0,6 M NaCl), 0,1% Natriumpyrophosphat und 25 mM Natriumphosphatpuffer (pH 7,0) 72 Stunden lang bei 65°C und einer Konzentration der Radioaktivität von 5 x 10⁶ cpm/ml durchgeführt. Die Filter wurden anschließend in 0,1% SDS, 2x SSC (300 mM Natriumchlorid, 30 mM Natrium₃-Citrat) bei einer Temperatur von 65°C gewaschen.

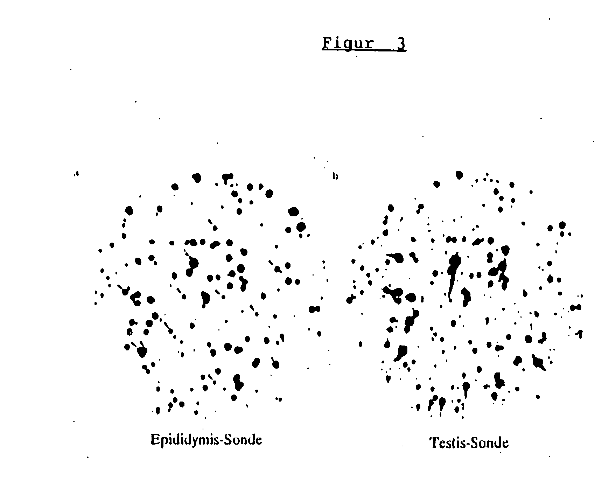

Die Figur 3 zeigt die Autoradiogramme zweier Replika-Filter von einer der Petrischalen, welche mit epididymalen (a) bzw. testikularen (b) cDNA-Sonden hybridisiert wurden. Die Entwicklung der Autoradiogramme erfolgte nach einer Expositionszeit von 16 Stunden unter Verwendung einer Verstärker-Folie. Die Fitter repräsentieren etwa 500 unabhängige Klone der im System Lambda gt 11 erstellten cDNA-Gesamtbibliothek aus Nebenhodengewebe. Die positiven. Epididymis-spezifischen Hybridisierungssignale sind mit Pfeilspitzen markiert (vgl. Figur 3a).

Mit Hilfe dieses Primär-Screening-Verfahrens (vgl. Figur 1a) konnten etwa 10 000 unabhängige cDNA-Klone-ana lysiert werden. Dabei konnten 265 Rekombinanten identifiziert werden, deren Signale nach Hybridisierung mit der epididymalen cDNA-Sonde im Vergleich zu der aus Hodengewebe hergestellten sehr viel stärker waren. Bezogen auf den durch Screening analysierten Teil der Phagen-Bibiothek entspricht die Anzahl positiver cDNA-Klone einem Anteil von 2,5%.

Positive cDNA-Klone wurden isoliert und für ein Sekundär-Screening in Gruppen mit bis zu 80 Klonen aufgeteilt.

Das oben beschriebene Verfahren wurde mit der Abweichung wiederholt, daß von jeder der in Gruppen von jeweils 80 Klonen bewachsenen Petrischalen je 3 Replika-Filter angefertigt wurden. Zur Herstellung der negativen cDNA-Sonden wurde hier Poly(A)⁺RNA aus menschlichem Gehirn bzw. aus menschlischer Leber (Clontech California, USA) verwendet.

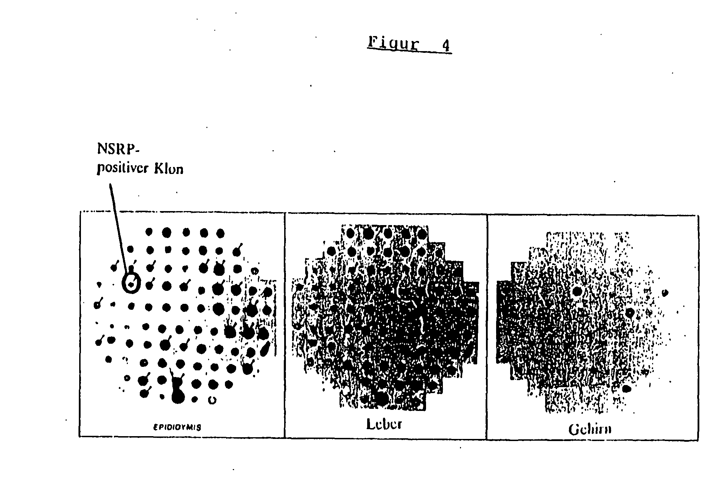

Die Anzahl positiver Rekombinanten wurde nach Analyse dieses sekundären Screening-Verfahrens (vgl. Figur 1 b) auf 59 Klone reduziert (vgl. Figur 4).

Die Isolierung positiver Klone erfolgte ohne weitere Reinigung der Plaques. Die gereinigte Lambda-DNA wurde mit EcoRI geschnitten und die Inserts durch Biotrap-Elution (Schleicher & Schüll) isoliert. Die EcoRI-Inserts wurden in den bakteriellen Plasmid-Vektor pBS (Stratagene, California, USA) subkloniert und durch das CaCl₂-Transformationsverfahren in die Bakterienzellen des E.coli-Stammes XL1 Blue (Stratagene, California) eingeführt (vgl. T Maniatis et al., Molecular cloning: A Laboratory Manual, Cold Spring Harbour Laboratory. Cold Spring Harbour, N.Y., (1982)).

### Beispiel 4

### Herstellung von Northern-Blots zur Analyse der gewebespezifischen Genexpression und Eintellung der Nebenhoden-spezifischen cDNA-Klone In Familien mit verwandten Sequenzen

20 µg Gesamt-RNA aus menschlichem Epididymis (E) von einem mit Cyproteronacetat behandelten Patienten sowie die gleiche Menge Gesamt-RNA aus menschlichem Hoden (T) und menschlicher Decidua (D) wurden in einem horizontalen 1,3%-igen Formldehyd-Agarosegel 16 Stunden lang bei einer konstanten Spannung von 25 Volt elektrophoretisch aufgetrennt (vgl. Maniatis et al., Molecular Cloning: A Laboratory Manual, Cold Spring Harbour Laboratory, Cold Spring Harbour, N.Y., (1982) (Figuren 5, 6 u. 7)). Zur Größenbestimmung der RNA's wurde das Gel zusätzlich mit einer RNA-Leiter (0,24-9,5 kB) beladen. Die RNA wurde anschließend durch Kapillar-Blotting in Gegenwart von 20 x SSC auf Nylonmembranen (Hybond N, Amersham) übertragen. Die Blots wurden nachfolgend mit radioaktiven, potentiell Epididymis-spezifischen cDNA-Sonden sukzessive hybridisiert. Die Sonden wurden durch EcoRI-Restriktion der im Plasmid pBS subklonierten Lambda gt11-DNA sowie anschließender Biotrap-Elution isoliert und wiesen nach radioaktiver Markierung eine spezifische Radioaktivität von > 10⁹ cpm/µg auf (vgl. A.P Feinberg und B. Vogelstein, A technique for radiolabelling DNA restriction endonuclease fragments to high specific activity, Analytical Biochemistry, 132, 6-13, (1983)). Die Hybridisierung erfolgte über Nacht bei einer Radioaktivitätskonzentration von 1-2 x 10⁶ cpm/ml. Nachfolgend wurden die Filter schrittweise unter steigender Stringenz gewaschen, beginnend mit 2 x SSC bei Raumtemperatur bis zu 0,1 x SSC bei einer Temperatur von 65°C. Zur Dehybridisierung wurden die Filter 15 Minuten bei 65°C in Gegenwart von 2 mM Tris (pH 7,5), 1 mM EDTA und 0,1% SDS inkubiert. Die getrockneten Filter wurden auf das Vorhandensein von Sondenmaterial autoradiographisch untersucht, bevor sie im Falle ausbleibender Signale für eine wiederholte Hybridisierung mit einer weiteren Sonde verwendet wurden.

In Figur 5 sind Beispiele für die Autoradiogramme der Northern-Blot-Analysen potentiell positiver cDNA-Klone dargestellt. Die Expositionszeit betrug 20 Stunden. Anhand der unterschiedlichen Hybridisierungsmuster wurden die Klone hinsichtlich ihrer Gewebespezifität qualitativ bewertet.
a) Positiver cDNA-Klon, angezeigt durch Nebenhoden-spezifische Hybridisierung.
b) Positives, aber nicht Nebenhoden-spezifisches Hybridisierungsmuster.
c) Positiver cDNA-Klon (*), der auch mit rRNA hybridisiert.
d) cDNA-Klon ohne Gewebe-spezifische Expression.

Mit Hilfe dieses Verfahrens konnte eine Anzahl von 36 epididymalen cDNA-Klonen identifiziert werden. Das spezifische Hybridisierungssignal eines jeden Klon-Inserts mit epididymaler RNA war um mindestens eine Größenordnung stärker als solche mit RNA aus Hoden oder Dezidua (vgl. Figur 5a).

Durch die Ergebnisse aus Kreuz-Hybridisierungen mit der ursprünglichen Phagenbibliothek konnten die 36 Nebenhoden-spezifischen cDNA-Klone sechs unabhängigen Familien zugeordnet werden, die jeweils einen Satz zueinander verwandter Klone umfaßten. Dabei konnte gezeigt werden, daß sich fünf der cDNA-Klon-Familien von unterschiedlichen, relativ kurzen mRNA-Molekülen mit einer durchschnittlichen Länge von 600 bis 1000 Nukleotiden ableiten, während der erfindungsgemäße Klon, der die genetische Information für das vorliegende Rezeptorprotein enthält, eine Länge von etwa 5000 Nukleotiden aufweist.

Da auch aus der zweiten cDNA-Bibliothek nur cDNA-Fragmente mit einer maximalen Länge von 3,7 kb isoliert werden konnten (vgl. Fig. 8b), aufgrund der Ergebnisse aus der Northern-Hybridisierung jedoch von einer mRNA-Länge von etwa 5 kb auszugehen war, wurde der 5'-Anfang dieser cDNA unter Anwendung der 5'-RACE-Methode kloniert (RACE = Rapid amplification of cDNA ends; M.A. Frohman et al., "Rapid production of full-length cDNAs from rare transcripts: Amplification using a single gene-specific oligonucleolide primer", Proc. Natl. Acad. Sci. USA, 85, S. 8998-9002 (1988)). Hierfür wurden aus dem 5'-Bereich der bekannten 3,7 kb umfassenden cDNA-Sequenz zwei genspezifische Antisense-Primer A1 und A2 (A1= 5'AGC TAT GGG AGC TGA AG 3'; A2= 5'TGT CAA TGG CAG GGC TG 3') hergestellt. Mit Hilfe dieser Primer wurde ausgehend von 1 µg Gesamt-RNA aus humanem Nebenhoden unter Anwendung des Protokolls "5'RACE System for Rapid Amplification of cDNA Ends" der Fa. Gibco BRL. Berlin, ein 5'-Fragment mit einer Länge von 996 Nukleotiden erhalten, welches mit der bereits bekannten cDNA-Sequenz über eine-Länge von 49 Nukleotiden (einschl. A1-Primer) überlappte (vgl. Fig. 8b, 5'RACE-Fragment, 1,0 kb).

### Beispiel 5

### Kontrolle der Gewebespezifität des erfindungsgemäßen Klons

Jeweils 20 µg Gesamt-RNA aus humaner Decidua, Nebenhoden (Epididymis), und Hoden (Testis) wurden gemäß Beispiel 4 aufgetrennt und auf Nylonmembranen übertragen. Zur Kontrolle der Gewebespezifität des erfindungsgemäßen Klons wurden außerdem jeweils 10 µg Poly(A)⁺RNA aus Nebenhoden und Gesamt-RNA aus Samenleiter (Vas deferens), sowie jeweils 20 µg Gesamt-RNA aus einer Lymphoblastoid-Zellinie (Daudi), aus Prostatakarzinom-Zellinien (LNCaP, DU145) und aus embryonalen, epididymalen Zellinien (RVP, REP) analog aufgetrennt und ebenfalls auf eine Nylonmembran übertragen. Als Sonde wurde in Figur 6a ein mit ³²P markiertes cDNA-Insert aus dem 3'-Bereich des erfindungsgemäßen Klons (vgl. Fig. 8b, 3'-UTR) eingesetzt und mit Northem-Blots gemäß Beispiel 4 hybridisiert. In Figur 6b wurde demgegenüber ein mit ³²P markiertes BamHI-cDNA-Insert aus dem 5'-Bereich des erfindungsgemäßen Klons eingesetzt (vgl. Fig 8b, 5'BamHI-Fragment). Die Figur 6c zeigt einen Northem-Blot nach Hybridisierung mit einem mit ³²P markierten 5'-RACE-Fragment des erfindungsgemäßen Klons.

Die in Figur 6 a-c dargestellten Ergebnisse zeigen, daß der erfindungsgemäß cDNA-Klon nur mit der epididymalen RNA ein eindeutiges Hybridisierungssignal liefert. Ferner konnte anhand des der Figur 6c entsprechenden Autoradiogramms die Echtheit des 5'-RACE-Fragments bestätigt werden (beachte das ausschließliche Signal mit Nebenhoden-RNA bei ca. 5 kb).

### Beispiel 6

### Nachweis homologer Sequenzen Im Nebenhoden anderer Säugetierarten und Kontrolle der Gewebespezifität des erfindungsgemäßen Klons in der Ratte

Um zu untersuchen, ob Homologe des erfindungsgemäßen Rezeptorproteins auch in anderen Säugetierarten mit gleicher Gewebespezifität exprimiert werden, wurde unter Anwendung der PCR-Technik (allg. Ref., vgl. R.S. Cha, und W.G. Thilly, Specificity, Efficiency and Fidelity of PCR, PCR-Methods and Applications, Vol. 3, S. 18 - 29 (1993)) versucht, mit dem humanen Subfragment E - F1 (vgl. Fig. 8b, PCR-Subklone, hergestellt unter Verwendung der zwei Primer E = 5' CAT CCG AAA ATA CAT CC 3' und F1 = 5'TGA AGG CAC ACA TCT CC 3') korrespondierende Fragmente aus Nabenhoden-Gesamt-RNA von Ratte, Maus und Hund zu amplifizieren.

Jeweils 5 µg Gesamt-RNA der genannten Tierarten wurden in einem Gesamtvolumen von je 20 µl unter Verwendung von 0,5 µg Oligo(dT)₁₂₋₁₈ (Pharmacia, Freiburg) einer Anelierung unterzogen. Die anschließende Erststrang-Synthese erfolgte in Gegenwart von je 10 mM dATP, dGTP, dCTP und dTTP sowie von 200 Einheiten reverser Transkriptase (Superscript, Gibco BRL, Berlin). Für die nachfolgende PCR-Amplifikation wurde jeweils 1 µl der Produkte der Erststrang-Synthese mit je 10 mM der vier dNTP's, je 20 pM der humanen genspezifischen Primer E und F1 (s. o.) sowie mit je 7 Einheiten Taq-Polymerase (Promega, Heidelberg) in einem Gesamtvolumen von je 50 µl vermischt und folgenden PCR-Zyklen unterworfen: Denaturierung (5 Minuten bei 95°C); dann 30 Zyklen umfassend Denaturierung (1 Minute bei 95°C), Anelierung (1 Minute bei 55°C) und Elongation (1 Minute bei 72°C). Die auf diese Weise erhaltenen komplementären cDNA-Fragmente wurden in 1,2 %igen Agarosegelen elektrophoretisch aufgetrennt. Anschließend wurden Fragmente mit einer Länge von etwa 750 Nukleotiden elektroeluiert und in den Klonierungsvektor pCRII (Invitrogen, ITC Biotechnology, Heidelberg) inseriert. Die Sequenzbestimmung der klonierten DNA-Fragmente erfolgte wie im folgenden Beispiel 7 beschrieben und ergab - sowohl auf der Nukleotid- als auch auf der Aminosäureebene - eine jeweilige Homologie von etwa 90 % zur humanen Sequenz. Dieses überraschende Ergebnis zeigt, daß die für das erfindungsgemäße Rezeptorprotein kodierende DNA-Sequenz zumindest unter Säugern ein hohes Maß an Konservierung aufweist.

Für die Kontrolle der Gewebespezifität des erfindungsgemäßen Ktons in der Ratte wurden jeweils 20 µg Gesamt-RNA aus verschiedenen Rattengeweben (mit Ausnahme der 2. Spur (Testis), nur 4 µg) gemäß Beispiel 4 aufgetrennt und auf Nylonmembranen übertragen. Als Sonde wurde ein mit ³²P markiertes cDNA-Insert (spez. Aktivität 19⁹ cpm/µg) aus dem Rattenhomologen des erfindungsgemäßen Klons (E - F1) eingesetzt und mit Northem-Blots gemäß Beispiel 4 hybridisiert In Figur 7 sind die nach einer Expositionszeit von 15 Stunden entwickelten Autoradiogramme der Northem-Blot-Analyse für den erfindungsgemäßen Klon schematisch dargestellt. In der Mitte der Figur 7 ist eine Rehybridisierung des darüber abgebildeten Blots mit einer Kontroll-Aktin-Sonde dargestellt, aus der hervorgeht, daß sich in allen Spuren des Gels in etwa äquimolare Mengen an RNA befunden haben.

Die Figur 7 zeigt, daß die Sonde E - F1 der Ratte ausschließlich mit Nebenhoden-RNA hybridisierte, wobei die Signale innerhalb des Nebenhodens im Caput (proximale Region) und in der Cauda (distale Region) am stärksten waren (vgl. Spuren 5-7).

Die Ergebnisse der Ratte bestätigen das Nebenhoden-spezifische Expressionsmuster der erfindungsgemäßen Sequenz, denn bei den anderen untersuchten Gewebearten (insgesamt 15) konnte keine Kreuzhybridisierung nachgewiesen werden.

### Beispiel 7

### Bestimmung der Nukleotid- und der abgeleiteten Aminosäuresequenz des erfindungsgemäßen cDNA-Klons

Die Basensequenz des längsten erfindungsgemäßen cDNA-Klons gemäß den Beispielen 4, 5 und 6 wurde mit Hilfe der Dideoxy-Methode nach Sanger, F. und Coulson, A.R. "A rapid method for determining sequences in DNA by primed synthesis with DNA polymerase" J. Mol. Biol. 94, 441 (1975) ermittelt. Zu diesem Zweck wurden Subklone gemäß Beispiel 3 hergestellt und unter alkalischen Bedingungen in Einzelstrang-DNA's überführt.

Das Ergebnis für den erfindungsgemäßen Klon ist im Sequenzprotokoll (SEQ ID NO 1) dargestellt.

Die Figur 8a zeigt das Ergebnis des Hydrophobizitäts-Plots, wobei hydrophobe Aminosäuresequenzen unterhalb der Null-Linie dargestellt sind. Auffällig sind die hydrophoben Bereiche zwischen Position 620 und Position 900 der Aminosäuresequenz, die die 7 Transmembrandomänen kennzeichnen und auf einen Rezeptor mit der in Figur 8c allgemein dargestellten Struktur (aus: J.D. Watson et al., "Rekombinierte DNA", 2. Auflage, Spektrum Verlag, S. 299 (1993)) schließen lassen. Beachtenswert ist ferner der 620 Aminosäuren umfassende extrazelluläre N-Terminus. In Figur 8b ist ein korrespondierendes Balkendiagramm des erfindungsgemäßen Proteins dargestellt. Es zeigt den offenen Leserahmen (ORF) in Form eines Kastens mit einer Länge von 3114 Nukleotiden, was einer Kodierungskapazität von 1038 Aminosäuren (aa) entspricht. Die 7 Transmembrandomänen sind schraffiert hervorgehoben und korrespondieren mit den Ergebnissen der Hydrophobizitätsanalyse. Die Nukleotidsequenz von Position 3115 bis Position 4665 umfaßt den 3'nicht-translatierten Bereich (3'UTR) des erfindungsgemäßen Klons und entspricht in etwa dem aus der ersten cDNA-Bibliothek isolierten cDNA-Klon. Unterhalb des Balkendiagramms sind die beiden cDNA-Klone dargestellt, die aus den zwei cDNA-Bibliotheken des Nebenhodens (library 1/library 2) isoliert und sequenziert wurden. Schließlich sind darunter PCR-Subklone und die für ihre jeweilige Synthese eingesetzten Primer (A1, A2, E, F1) dargestellt. Die als Sonden verwendeten Fragmente sind als schwarze Balken hervorgehoben.

### Beispiel 8

### Herstellung von Antigenen über bakterielle Expressionsvektoren zur Induktion von Antikörpern

Der proteinkodierende Bereich des in den gemäß Beispiel 3 hergestellten pBS-Subklonen inserierten erfindungsgemäßen cDNA-Klons wurde mit Hilfe üblicher Restriktionsendonukleasen herausgeschnitten und auf üblichem Wege (Sambrook et al., "Molecular Cloning - A Laboratory Manual", 2. Auflage, Cold Spring Harbor Laboratory Press (1989)) in den Expressions-Plasmidvektor pET3 (Rosenberg et al., "Vectors for selective expression of cloned DNAs by T7 RNA Polymerase", Gene, 56, 125-135 (1987)) unter Berücksichtigung das Leserasters umkloniert. Dieser Vektor ermöglicht die Insertion des cDNA-Inserts direkt hinter (stromabwärts) eine für das Enzym T7-Polymerase spezifischen Promotorsequenz und umfaßt außerdem ein Ampicillin-Resistenzgen.

Nach Transformation dieser Konstrukte in den E. coli Stamm BL21 (DE3), welcher über das Gen für die T7-Polymerase verfügt, erfolgte die Anzucht der Transformanten unter Ampicillinselektion. Durch Zugabe von Rifampicin wurden alle E. coli eigenen Polymerasen inaktiviert. Da lediglich die T7-Polymerase gegen dieses Antibiotikum resistent ist und das erfindungsgemäße cDNA-Insert der Kontrolle des Promotors für die T7-Polymerase unterstand, wurde primär das erfindungsgemäße Polypeptid exprimiert.

Die auf diese Weise hergestellten bakteriogenen Polypeptide wurden anschließend aus den Bakterienextrakten gelelektrophoretisch aufgereinigt und direkt als Antigene für eine Immunisierung von Kaninchen, Hühnern und Ratten verwendet.

### Beispiel 9

### Herstellung von Antigenen über chemische Synthese zur induktion von Antikörpern

Die Sequenz Arg-Ile-Lys-Lys-Lys-Lys (korrespondierend mit den Nukleotiden 2503-2520 bzw. Codons 835-840 der SEQ ID NO 1) wurde per Computer als mögliches antigenes Epitop des erfindungsgemäßen Rezeptorproteins identifiziert und zusammen mit den flankierenden Aminosäuren in der Gesamtsequenz Cys-Arg-Ile-Lys-Lys-Lys-Lys-Gln-Leu-Gly-Ala-Gln-Arg-Lys-Thr (korrespondierend mit den Nukleotiden 2500-2544 bzw Codons 834-848 der SEQ ID NO 1) nach dem Verfahren von Merrifield (J.M. Stewart, "Synthesis and use of neuropeptides" Neuroendocrine Peptide Methodology, ed. P.M. Conn, Academic Press, NewYork, S. 815-844 (1989)) chemisch synthetisiert, wobei ein natürliches Cystein N-terminal enthalten ist.

Die auf diese Weise hergestellten immunogenen Fragmente des erfindungsgemäßen Polypeptids wurden anschließend jeweils mit m-Maleimidobenzoicsäure-N-hydroxysuccin imid-ester (M8759, Sigma) (Sambrook et aL, "Molecular Cloning - A Laboratory Manual", 2. Auflage, Cold Spring Harbor Laboratory Press (1989)) an Trägersubstanzen (Keyhole-Limpet-Hämocyanin, KLH) gekoppelt und als Antigene für die Immunisierung von Kaninchen, Hühnem und Ratten zur Erzeugung von Antikörpern eingesetzt.

Die so erhaltenen polyklonalen Antikörper sind insbesondere für den qualitativen und quantitativen diagnostischen Nachweis des erfindungsgemäßen Rezeptorproteins in Gewebeschnitten von Infertilitätspatienten geeignet. Der Nachweis kann nach bekannten immunhistologischen Methoden beispielsweise durch Verwendung eines ggfs. nach üblichen Verfahren markierten, im Handel erhältlichen zweiten Antikörpers erfolgen, welcher den erstgenannten Antikörper in spezifischer Welse erkennt (Harlow und Lane, Antibodies - A Laboratory Manual, Cold Spring Harbour Laboratory Press, Cold Spring Harbour, New York (1988)).

### Beispiel 10

### In situ Transkript-Hybridisterung der erfindungsgemäße mRNA In Nebenhoden-Gewebeschnitten von Mensch und Rette

Unter Verwendung des 743 Bp langen Fragments E - F1 (vgl. Bsp. 6) der erfindungsgemäßen DNA-Sequenz wurde in Gegenwart von [α³⁵S]CTP durch in-vitro Transkription eine spezifische Sonde hergestellt, die zur Bestimmung der Expression der erfindungsgemäßen Sequenz in Gefrierschnitten eingesetzt wurde.

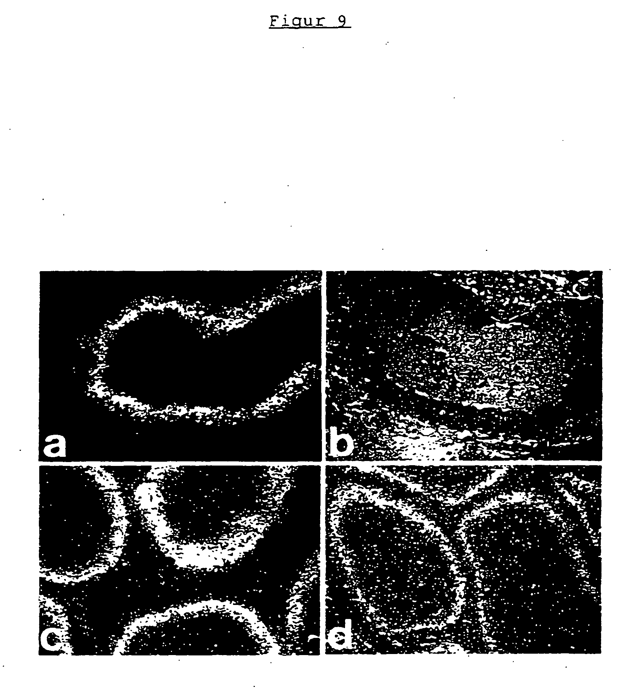

Die Ergebnisse entsprechender in situ-Hybridisierungsexperimente sind in Figur 9 dargestellt. Die weißen Pünktchen des in Bild a unter Verwendung eines Dunkelfeld-Mikroskops dargestellten Gefrierschnitts humanen Ursprungs zeigen spezifische Signale aus der in situ-Hybridisierung in Gegenwart des obigen Fragments als "Antisense" -Sonde. Bild b repräsentiert das Ergebnis eines mit einem vergleichbaren Gewebeschnitt durchgeführten Kontrollversuchs, bei dem anstelle der zuvor verwendeten "Antisense"-Sonde der komplementäre Strang als "Sense"-Sonde verwendet wurde (Nagativ-Kontrolle).

Das Bild a zeigt deutlich, daß die erfindungsgemäße DMA-Sequenz ausschließlich in der Epithelschicht des Nebenhodens, nicht jedoch in den Stroma- oder Muskelzellen des gleichen humanen Gewebes exprimiert wird.

Die Bilder c und d zeigen parallel durchgeführte Versuche unter Verwendung von Gewebeschnitten des Ratten-Nebenhodens. Bild c zeigt einen Schnitt der proximalen Region des Nebenhodens, während in Bild d die distale Region des gleichen Gewebes dargestellt ist. Bei vergleichender Betrachtung der Bilder c und d wird deutlich, daß die proximale Region stärkere Signale liefert, was vermutlich auf eine funktionelle Spezialisierung dieses Bereichs zurückzuführen ist.

Die Ergebnisse dieses Beispiels belegen die extrem hohe Zeiltypspezifität des erfindungsgemäßen Rezeptorproteins und demgemäß die Eignung der erfindungsgemäß vorgeschlagenen Substanzen zur spezifischen und damit ausschließlichen positiven oder negativen Beeinflussung der Funktionen des Nebenhodens im Zusammenhang mit der Spermienreifung. Ferner veranschaulichen die Ergebnisse die erfindungsgemäß vorgeschlagene Verwendung der bereitgesteliten Sequenzen einschließlich Fragmente derselben als spezifische Sonden für sowohl qualitative als auch quantitative diagnostische Untersuchungen von Biopsieproben. Überdies weist die überaus hohe Expressionsrate des erfindungsgemäßen Rezeptorproteins auf dessen Eignung als erfolgversprechender Mediator im Rahmen therapeutischer oder kontrazeptiver Anwendungsformen hin.

### Beispiel 11

### Verwendung des erfindungsgemäßen Rezeptorproteins bzw. der dafür kodierenden cDNA zur isolierung spezifischer Liganden

Zur Gewinnung spezifischer Uganden für das erfindungsgemäße Rezeptorprotein wurde die N-terminale extrazelluläre Domäne (von Position 1 bis 620 der Aminosäuresequenz gemäß SEQ ID NO 2) des Rezeptorproteins in einem eukaryontischen Expressionssystem wie der Zellinie COS-7 hergestellt. Zu diesem Zweck wurde der für diese Domäne kodierende Bereich der cDNA (entsprechend Position 1 bis 1860 der Nukleotidsequenz gemäß SEQ ID NO 1) am 3'-Ende mit einer Flag-Sequenz, d.h. einer Oligonukleotidsequenz versehen, die für ein bekanntes, hochspezifisches Peptidepitop kodiert, und in den Expressionsvektor pRc/CMV (Invitrogen, San Diegeo, Californien, USA) kloniert. Nach erfolgter Transfektion der Zellinie COS-7 mit dem beschriebenen Expressionsvektor wurde das Fusionsprodukt nach bekannten Verfahren gewonnen und durch Affinitätschromatographie unter Verwendung immobilisierter, gegen das Flag-Epitop gerichteter Antikörper gereinigt.

Anschließend wurde das Fusionsprodukt als Sonde in einem konventionellen Protein Screening-Verfahren (vgl. J. Sambrook, E.F. Fritsch, und T. Maniatis, Molecular Cloning: A Laboratory Manual, Cold Spring Harbor Laboratory, Cold Spring Harbor, N.Y., Kapitel 2 (1989)) unter Verwendung einer In Lambda-Bakteriophagen hergestellten cDNA-Expressionsbibliothek aus humanem Hoden eingesetzt. Hierzu wurden etwa 1 Million unabhängige cDNA-Bakterienklone, die durch IPTG-Induktion testikuläre Produkte und somit potentielle Liganden exprimieren, auf Nytonmambtanfiltertransteriert und unter geeigneten Bedingungen (s.o.) mit der rekombinanten Rezeptor-Bindungsdomäne inkubiert. Nachfolgend wurden die nicht spezifisch gebundenen Rezeptorffagmente unter stringenten Waschbedingungen entfernt, so daß sich auf den Filtern lediglich spezifische Bindungskomplexe befanden, die mit konventionellen Antikörper-Detektionssystemen (System unter Verwendung alkalischer Phosphatase, Sigma. Delsenhofen) über das Flag-Epitop sichtbar gemacht werden kannten. Die auf diese Weise identifizierten Phagenkolonien wurden isoliert und gereinigt sowie einer Sequenzbestimmung zugeführt.

Um zu ermitteln, ob die aufgefundenen Liganden eine Signaltransduktion durch den erfindungsgemäßen Rezeptor induzieren können und daher zur Stimulierung der Spermienreifung bei subfertilen Säugern geeignet sind, wurden wurden Kulturen der mit dem gesamten cDNA-Konstrukt transfizierten COS-7-Zellinie getrennt mit den positiven Liganden inkubiert und deren durch die spezifische Bindung verursachte Veränderung des intrazellulären cAMP-Spiegels und/ oder des Ca²⁺-Gehalts ermittelt. Positive Liganden, die nicht in der Lage waren, derartige Veränderungen hervorzurufen, sind beispielsweise als Antagonisten zur Inhibition der Spermienreifung geeignet und können zur Herstellung kontrazeptiver Mittel eingesetzt wenden.

Die Verwendung derartiger Liganden für therapeutische Zwecke ist besonders vorteilhaft, da durch die gewebe-spezifische Expression des erfindungsgemäßen Rezeptorproteins dieser Gewebetyp selektiv beeinflußt wird und daher keine oder nur geringe Nebenwirkungen zu erwarten sind.

## Patentansprüche

1. Nukleinsäure umfassend
a. die in Seq ID NO 1 dargestellten Nukleotidsequenz,
b. eine Teilsequenz der in a. angegebenen Sequenz von den Nukleotiden 1 bis 3114,
c. zu den vorgenannten Sequenzen homologe Sequenzen mit einem Homologiegrad von mindestens 90%,
d. eine einer Sequenz aus a. und b. im Rahmen der Degeneration des genetischen Codes entsprechenden Nukleotidsequenz oder
e. eine mit den Sequenzen aus a., b. oder d. unter stringenten Bedingungen hybridisierende Nukleotidsequenz,
wobei die Nukleinsäure von a - e für ein Nebenhoden spezifisches Rezeptorprotein kodiert.

2. Nukleinsäure nach Anspruch 1 umfassend einen Protein-kodierenden Abschnitt der der in Seq ID NO 1 dargestellten Nukleinsäuresequenz entspricht.

3. Nukleinsäure, kodierend für ein Polypeptid mit der in Seq ID NO 2 dargestellten Aminosäuresequenz.

4. Polypeptide kodiert von einer Nukleinsäure nach einem der Ansprüche 1-3.

5. Polypeptid, umfassend die in Seq ID NO 2 dargestellte Aminosäuresequenz.

6. Polypeptidfragment entsprechend der Aminosäuresequenz gemäß Seq ID NO 2 von Position 1 bis 620.

7. Antikörper, **dadurch gekennzeichnet, dass** sie in der Lage sind, eine Immunreaktion mit einem der Proteine oder Polypeptide gemäß Anspruch 4-6 einzugehen.

8. Monoklonaler Antikörper gemäß Anspruch 7.

9. Vektor-Moleküle enthaltend mindestens eine Kopie einer Nukleinsäure nach einem der Ansprüche 1-3.

10. Vektor-Moleküle nach Anspruch 9, **dadurch gekennzeichnet**, das sie die Sequenzen in solcher Weise inseriert enthalten, dass deren Expression in geeigneten Mikroorganismen erfolgen kann.

11. Prokaryotische oder eukaryotische Wirtszelle, **dadurch gekennzeichnet, dass** sie transfiziert ist mit einer Nukleinsäure nach einem der Ansprüche 1-3 oder mit einem Vektor nach Anspruch 9.

12. Verfahren zur Herstellung der Proteine oder Polypeptide nach Anspruch 4-6 **dadurch gekennzeichnet, dass** die Wirtszellen nach Anspruch 11 unter Bedingungen kultiviert werden, welche die Expression der DNA-Sequenz erlauben, und das Expressionsprodukt aus dem Kulturansatz gewonnen werden kann.

13. Pharmazeutische Zusammensetzung, welche eines oder mehrere der Proteine oder Polypeptide gemäß Anspruch 4-6 oder hergestellt nach Anspruch 12 oder eine Zelle nach Anspruch 11 als aktive Komponente enthält.

14. Pharmazeutische Zusammensetzung, welche Antikörper nach Anspruch 7-8 als aktive Komponente enthält.

15. Pharmazeutische Zusammensetzung, welche Nukleotidsequenzen als aktiven Wirkstoff enthält, die mit den Nukleotidsequenzen nach den Ansprüchen 1-3 hybridisieren.

16. Pharmazeutische Zusammensetzung gemäß Anspruch 15, wobei die Nukleotidsequenzen mit einem nachweisbaren Marker versehen sind.

17. Pharmazeutische Zusammensetzung, nach den Ansprüchen 13-16 zur Diagnose männlicher Fortpflanzungsstörungen.

18. Pharmazeutische Zusammensetzung, nach den Ansprüchen 13-16 zur Therapie männlicher Fortpflanzungsstörungen oder Kontrazeption.

19. Verwendung der Proteine oder Polypeptide gemäß Anspruch 4-6 zur Isolierung und Herstellung von spezifischen Liganden.

## Claims

1. Nucleic acid comprising
a. the nucleotide sequence depicted in SEQ ID No. 1,
b. a partial sequence of the sequence indicated in a. from nucleotides 1 to 3114,
c. sequences homologous to the aforementioned sequences and having a degree of homology of at least 90%,
d. a nucleotide sequence corresponding to a sequence from a. and b. within the scope of the degeneracy of the genetic code or
e. a nucleotide sequence hybridizing with the sequences from a., b. or d. under stringent conditions,
where the nucleic acid of a-e codes for an epididymis-specific receptor protein.

2. Nucleic acid according to Claim 1, comprising a protein-encoding section which corresponds to the nucleic acid sequence depicted in SEQ ID No. 1.

3. Nucleic acid coding for a polypeptide having the amino acid sequence depicted in SEQ ID No. 2.

4. Polypeptides encoded by a nucleic acid according to any of Claims 1-3.

5. Polypeptide comprising the amino acid sequence depicted in SEQ ID No. 2.

6. Polypeptide fragment corresponding to the amino acid sequence shown in SEQ ID No. 2 from position 1 to 620.

7. Antibodies **characterized in that** they are able to enter into an immune reaction with one of the proteins or polypeptides according to Claim 4-6.

8. Monoclonal antibody according to Claim 7.

9. Vector molecules comprising at least one copy of a nucleic acid according to any of Claims 1-3.

10. Vector molecules according to Claim 9, **characterized in that** they comprise the sequences inserted in such a way that expression thereof can take place in suitable microorganisms.

11. Prokaryotic or eukaryotic host cell, **characterized in that** it is transfected with a nucleic acid according to any of Claims 1-3 or with a vector according to Claim 9.

12. Method for preparing the proteins or polypeptides according to Claim 4-6, **characterized in that** the host cells according to Claim 11 are cultivated under conditions which permit expression of the DNA sequence, and the expression product can be obtained from the culture mixture.

13. Pharmaceutical composition which comprises one or more of the proteins or polypeptides according to Claim 4-6 or prepared according to Claim 12 or a cell according to Claim 11 as active component.

14. Pharmaceutical composition which comprises antibodies according to Claim 7-8 as active component.

15. Pharmaceutical composition which comprises nucleotide sequences which hybridize with the nucleotide sequences according to Claims 1-3 as active ingredient.

16. Pharmaceutical composition according to Claim 15, where the nucleotide sequences are provided with a detectable marker.

17. Pharmaceutical composition according to Claims 13-16 for diagnosing male reproductive impairments.

18. Pharmaceutical composition according to Claims 13-16 for therapy of male reproductive impairments or contraception.

19. Use of the proteins or polypeptides according to Claim 4-6 for the isolation and preparation of specific ligands.

## Revendications

1. Acide nucléique comportant
a. la séquence nucléotidique représentée dans SEQ ID NO 1,
b. une séquence partielle de la séquence indiquée sous a des nucléotides 1 à 3114,
c. des séquences homologues aux séquences précitées avec un degré d'homologie d'au moins 90 %,
d. une séquence nucléotidique correspondant à une séquence de a. et b. dans le cadre de la dégénération du code génétique, ou
e. une séquence nucléotidique qui s'hybride dans des conditions rigoureuses avec les séquences de a., b. ou d.,
l'acide nucléique de a-e codant pour une protéine réceptrice spécifique d'épididyme.

2. Acide nucléique suivant la revendication 1, comportant un segment codant pour une protéine de la séquence d'acide nucléique représentée dans SEQ ID NO 1.

3. Acide nucléique, codant pour un polypeptide présentant la séquence d'acides aminés représentée dans SEQ ID NO 2.

4. Polypeptide codé par un acide nucléique suivant l'une des revendications 1 à 3.

5. Polypeptide comprenant la séquence d'acides aminés représentée dans SEQ ID NO 2.

6. Fragment de polypeptide correspondant à la séquence d'acides aminés suivant SEQ ID NO 2 de la position 1 à 620.

7. Anticorps **caractérisé en ce qu'**ils sont en état de donner lieu à une réaction immunitaire avec une des protéines ou polypeptides suivant les revendications 4 à 6.

8. Anticorps monoclonal suivant la revendication 7.

9. Molécules de vecteur contenant au moins une copie d'un acide nucléique suivant l'une des revendications 1 à 3.

10. Molécules de vecteur suivant la revendication 9, **caractérisées en ce qu'**elles contiennent les séquences à l'état inséré de telle manière que leur expression peut avoir lieu dans des micro-organismes appropriés.

11. Cellule hôte procaryote ou eucaryote, **caractérisée en ce qu'**elle est transfectée avec un acide nucléique suivant l'une des revendications 1 à 3 ou avec un vecteur suivant la revendication 9.

12. Procédé de préparation des protéines ou polypeptides suivant les revendications 4 à 6, **caractérisé en ce que** les cellules hôte suivant la revendication 11 sont cultivées dans des conditions qui permettent l'expression de la séquence d'ADN et **en ce que** le produit d'expression peut être obtenu à partir de la masse de culture.

13. Composition pharmaceutique, qui contient un ou plusieurs des protéines ou polypeptides suivant les revendications 4 à 6 ou préparés selon la revendication 12 ou une cellule selon la revendication 11, comme composant actif.

14. Composition pharmaceutique, qui contient, comme composant actif, des anticorps selon les revendications 7 et 8.

15. Composition pharmaceutique, qui, comme substance active, contient des séquences nucléotidiques qui s'hybrident avec les séquences nucléotidiques selon les revendications 1 à 3.

16. Composition pharmaceutique suivant la revendication 15, dans laquelle les séquences nucléotidiques sont pourvues d'un marqueur décelable.

17. Composition pharmaceutique suivant les revendications 13 à 16, pour le diagnostic de troubles de la reproduction chez l'homme.

18. Composition pharmaceutique suivant les revendications 13 à 16, pour la thérapie de troubles de la reproduction chez l'homme ou pour la contraception.

19. Utilisation des protéines ou polypeptides suivant les revendications 4 à 6, pour l'isolement et la préparation de ligands spécifiques.
